# EUROPEAN PATENT SPECIFICATION

(11) **EP 4 531 810 B1**
(45) Date of publication and mention of the grant of the patent: **28.10.2026**
(21) Application number: 23727581.3
(22) Date of filing: 22.05.2023
(51) Int. Cl.: A61K 9/08, A61K 9/00, A61K 47/02, A61K 47/06, A61K 47/10, A61K 47/12, A61K 47/14, A61K 47/20, A61K 47/22, A61K 47/26, A61K 47/32, A61K 47/34, A61K 47/36, A61K 47/38, A23K 20/121, A23K 20/111, A23K 20/132, A23K 50/10, A61P 1/00

(54) **AQUEOUS PHARMACEUTICAL COMPOSITIONS COMPRISING SGLT-2 INHIBITORS**
WÄSSRIGE PHARMAZEUTISCHE ZUSAMMENSETZUNGEN, DIE SGLT-2-INHIBITOREN UMFASSEN
COMPOSITIONS PHARMACEUTIQUES AQUEUSES CONTENANT DES INHIBITEURS SGLT-2

(30) Priority: 25.05.2022 EP 22175413
(43) Date of publication of application: 09.04.2025
(73) Proprietor: Boehringer Ingelheim Vetmedica GmbH, 55216 Ingelheim am Rhein (DE)
(72) Inventor: DUCH, Thomas, Adam, 55216 Ingelheim am Rhein (DE); WEILER, Claudius, 55216 Ingelheim am Rhein (DE)
(74) Representative: Boehringer Global Patents
(86) International application number: PCT/EP2023/063549
(87) International publication number: WO 2023/227492

(56) References cited:
- EP-A1- 1 609 785
- WO-A1-2008/116179
- WO-A1-2015/173584
- WO-A1-2017/032799
- WO-A1-2021/078964
- WO-A2-2006/064033
- CN-A- 111 870 694
- US-A1- 2011 077 213
- US-A1- 2017 145 000
- US-A1- 2020 237 794
- US-A1- 2022 016 078

## Description

### FIELD OF THE INVENTION

The invention relates to the field of medicine, particularly veterinary medicine. In particular, the invention relates to novel aqueous pharmaceutical compositions comprising at least one SGLT-2 inhibitor and one or more solubilizing agents.

### BACKGROUND OF THE INVENTION

The treatment of diabetes and other metabolic disorders includes the inhibition of the renal sodium-dependent glucose co-transporter SGLT-2. SGLT-2 in the kidney regulates glucose levels by mediating the reabsorption of glucose back into the plasma following filtration of the blood. SGLT-2 inhibition thus induces glucosuria and may reduce blood glucose levels.

A large variety of SGLT-2 inhibitors are known. A pharmaceutical formulation of SGLT-2 inhibitors is essential in order to administer such compounds in an adequate way to the patient.

EP 1 609 785 discloses C-glycoside derivatives and salts thereof, wherein B ring is bonded to A ring via -X- and A ring is directly bonded to the glucose residue, and it is usable as a Na⁺-glucose cotransporter inhibitor, especially for a therapeutic and/or preventive agent for diabetes such as insulin-dependent diabetes (type 1 diabetes) and insulin-independent diabetes (type 2 diabetes), as well as diabetes related diseases such as an insulin-resistant diseases and obesity.

WO 2006/064033 discloses glucopyranosyl-substituted benzene derivatives including the tautomers, the stereoisomers thereof, the mixtures thereof and the salts thereof, which are suitable for the treatment of metabolic disorders.

SGLT-2 inhibitors are for instance described in WO 2007/028814 which is directed to crystalline forms of 1-chloro-4-([beta]-D-glucopyranos-1-yl)-2-(4-ethynyl-benzyl)-benzene, a method for the preparation thereof, as well as the use thereof for preparing medicaments. It discloses solutions of 1-chloro-4-([beta]-D-glucopyranos-1-yl)-2-(4-ethynyl-benzyl)-benzene in a solvent or a mixture of solvents and further specifies exemplarily suitable organic solvents such as ethanol or ethanol/water mixtures.

WO 2007/080170 describes crystalline forms of 1'-(1-methylethyl)-4'-[(2-fluoro-4-methoxyphenyl) methyl]-5'-methyl-1H-pyrazol-3'-O-[beta]-D-glucopyranoside, a method for the preparation thereof, as well as the use thereof for preparing medicaments. It discloses solutions of 1'-(1-methylethyl)-4'-[(2-fluoro-4-methoxyphenyl) methyl]-5'-methyl-1H-pyrazol-3'-O-[beta]-D-glucopyranoside in a solvent or a mixture of solvents and further specifies exemplarily suitable organic solvents such as ethanol or ethanol/water mixtures.

In addition, WO 2007/093610 describes glucopyranosyl-substituted benzonitrile derivatives, pharmaceutical compositions containing such compounds, their medical uses as well as processes for their manufacture. It mentions that such glucopyranosyl-substituted benzonitrile derivatives can be formulated among other with one or more inert carriers and/or diluents, such as water/ethanol, water/glycerol, propylene glycol and the like. It further discloses among many other compounds also 1-cyano-2-(4-cyclopropyl-benzyl)-4-(β-D-glucopyranos-1-yl)-benzene (velagliflozin).

Further SGLT-2 inhibitors are described in WO 2007/128749 which relates to glucopyranosyl-substituted benzonitrile derivatives, pharmaceutical compositions containing such compounds, their medical uses as well as processes for their manufacture. It mentions that such glucopyranosyl-substituted benzonitrile derivatives can be formulated among other with one or more inert carriers and/or diluents, such as water/ethanol, water/glycerol, propylene glycol and the like. It further discloses among many other compounds also 1-cyano-2-(4-cyclopropyl-benzyl)-4-(β-D-glucopyranos-1-yl)-benzene (velagliflozin).

US 2011/077213 discloses methods for treating and/or preventing kidney stones, employing an SGLT-2 inhibitor alone, or in combination with a supply of carbohydrate, and/or in combination with a diuretic agent.

WO 2013/079501 is directed to crystalline dapagliflozin hydrate and a method for the preparation thereof. It discloses solutions of dapagliflozin in a solvent or a mixture of solvents and further specifies exemplarily suitable solvents such as water and C1-C4 alcohols or mixtures thereof.

WO 2014/016381 (US 2014/031540) describes crystalline complexes of 1-cyano-2-(4-cyclopropyl-benzyl)-4-([3-D-glucopyranos-1-yl)-benzene with natural amino acids, methods for the preparation thereof as well as the use thereof for preparing medicaments. It discloses solutions of 1-cyano-2-(4-cyclopropyl-benzyl)-4-(β-D-glucopyranos-1-yl)-benzene in a solvent or a mixture of solvents and further specifies exemplarily suitable organic solvents such as C1-C4 alkanols, ethanol and mixtures thereof, in particular with water.

Furthermore, WO 2014/195966 describes amorphous forms of canagliflozin and processes of manufacturing thereof as well as corresponding pharmaceutical compositions and their medicinal uses. It discloses solutions of canagliflozin in one or more organic solvents and further specifies exemplarily suitable organic solvents such as ethanol.

WO 2015/110402 relates to SGLT-2 inhibitors for use in the treatment and/or prevention of metabolic disorders in canine animals.

WO 2015/173584 discloses methods for avoiding an increase in glucagon secretion associated with the administration of a sodium glucose co-transporter 2 (SGLT-2) inhibitor via the co-administration of a dipeptidyl peptidase IV (DPP IV) inhibitor. Additionally, methods are disclosed for normalizing the glucagon secretion associated with the administration of a sodium glucose co-transporter 2 (SGLT-2) inhibitor via the co-administration of a dipeptidyl peptidase IV (DPP IV) inhibitor. The WO publication also relates to methods for treating diabetes, especially Type 2 diabetes, as well as hyperglycemia, hyperinsulinemia, obesity, hypertriglyceridemia, Syndrome X, diabetic complications, atherosclerosis and related diseases, comprising administering an SGLT-2 inhibitor and a dipeptidyl peptidase IV (DPP IV) inhibitor.

WO 2017/032799 deals with liquid pharmaceutical compositions comprising at least one SGLT-2 inhibitor and one or more polar organic solvents, wherein the one or more polar organic solvents preferably always comprise propane-1,2-diol (propylene glycol).

US 2017/145000 discloses canagliflozin monohydrate and its crystalline forms.

US 2020/237794 discloses one or more SGLT-2 inhibitors or pharmaceutically acceptable forms thereof for use in the treatment and/or prevention of a metabolic disorder of an equine animal, such as laminitis, vascular dysfunction, hypertension, hepatic lipidosis, atherosclerosis, hyperadrenocorticism, Pituitary Pars Intermedia Dysfunction and/or Equine Metabolic Syndrome.

WO 2020/219645 discloses methods of reducing the weight loss and/or increasing the weight of a feline in need thereof and/or methods of managing the disease of a feline with diabetes mellitus and elevated IGF-1 concentration, said methods include administering to a feline in need thereof a total daily dosage of about 2 to 50 mg of velagliflozin or a pharmaceutically acceptable form thereof.

WO 2021/105152 discloses the use of at least one SGLT-2 inhibitor in a non-human mammal, preferably ruminant, preferably for drying-off of a non-human mammal, preferably ruminant, as well as corresponding methods, such as improving and/or facilitating the drying-off of a non-human mammal, preferably ruminant, comprising administering to such non-human mammal, preferably ruminant, at least one SGLT-2 inhibitor.

WO 2021/165177 discloses the use of one or more SGLT-2 inhibitors or pharmaceutically acceptable forms thereof for the prophylaxis and/or treatment of one or more cardiac diseases in feline animals.

US 2022/016078 discloses a method of treating or preventing kidney stones in a patient: a therapeutically effective amount of a compound or a pharmaceutically acceptable salt, solvate or hydrate thereof which induces glucosuria or a pharmaceutical composition thereof is administered to the patient in need thereof.

WO 2023/006718 discloses the use of one or more SGLT-2 inhibitors or pharmaceutically acceptable forms thereof for the prophylaxis and/or treatment of one or more cardiac diseases in a non-human mammal / non-human mammal patient excluding a feline, in particular a canine / canine patient.

WO 2023/006745 discloses the use of one or more SGLT-2 inhibitors or pharmaceutically acceptable forms thereof for the prophylaxis and/or treatment of hypertension in a non-human mammal, preferably a carnivore, in particular a cat or a dog.

WO 2023/006747 discloses the use of one or more SGLT-2 inhibitors or pharmaceutically acceptable forms thereof for the prophylaxis and/or treatment of one or more renal diseases in a non-human mammal, such as a carnivore, in particular a cat or a dog.

Xu G et al. (Journal of Medical Chemistry 2014, 57: 1236-1251) is directed to the design, synthesis and biological evaluation of deuterated C-aryl glycosides as potent and long-acting renal SGLT-2 inhibitors for the treatment of type 2 diabetes.

Further challenges known in the prior art are the limited solubility of SGLT-2 inhibitors in water due to their positive log₁₀P values, which typically influences the bioavailability in the body of a patient or makes it difficult to find adequate solvents to get the substance dissolved in a liquid formulation before administering it into the body of a patient.

### SUMMARY OF THE INVENTION

The present invention concerns an aqueous pharmaceutical composition comprising at least one SGLT-2 inhibitor and one or more solubilizing agents, wherein: (i) the at least one SGLT-2 inhibitor is velagliflozin, (ii) velagliflozin is the only SGLT-2 inhibitor contained in such aqueous pharmaceutical composition, (iii) such aqueous pharmaceutical composition is substantially free of organic solvents, and (iv) the one or more solubilizing agents are selected from the group consisting of: surfactants, anionic surfactants, non-ionic surfactants, hydrogenated castor oils, polyoxyethylene-polyoxypropylene block copolymers, polyethylene glycols, propylene glycol derivatives, polyvinylpyrrolidones.

In the course of the present invention the term "aqueous" with regard to the term "pharmaceutical composition" refers to a water-containing pharmaceutical composition, wherein preferably such water is present in the form of an aqueous buffer, such as for instance citric acid buffer. However, any suitable aqueous buffer (system) can be used according to the knowledge and skills of the person skilled in the art. The water content / aqueous buffer content of such water-containing pharmaceutical composition is from 30 to 100 g/100 mL (30 to 100 % w/v), preferably from 30 to 95 g/100 mL (30 to 95 % w/v), more preferably from 30 to 90 g/100 mL (30 to 90 % w/v), more preferably from 30 to 85 g/100 mL (30 to 85 % w/v), even more preferably from 30 to 80 g/100 mL (30 to 80 % w/v), most preferably from 50 to 80 g/100 mL (50 to 80 % w/v).

Velagliflozin, which is the SGLT-2 inhibitor comprised in the aqueous pharmaceutical composition of the invention, is represented by formula (2):

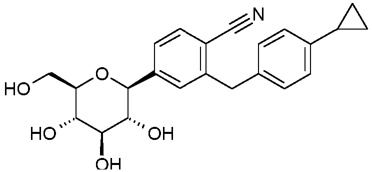

The present invention also concerns an aqueous pharmaceutical composition as described and/or claimed herein, wherein the aqueous pharmaceutical composition is for, preferably direct, administration to a subject, preferably without further mandatory processing and/or purification steps, more preferably an animal, even more preferably a mammal, in particular a horse, cat, dog or cow; wherein preferably the aqueous pharmaceutical composition is sterile.

The present invention also concerns an aqueous pharmaceutical composition as described and/or claimed herein for use in a method of treating and/or preventing one or more medicinal indications in a subject in need of such treatment and/or prevention, preferably an animal, more preferably a mammal, in particular a horse, cat, dog or cow, selected from among the medicinal indications:
(i) a metabolic disorder of an equine animal, wherein preferably the metabolic disorder is one or more disorders selected from insulin resistance, hyperinsulinemia, impaired glucose tolerance, dyslipidemia, dysadipokinemia, subclinical inflammation, systemic inflammation, low grade systemic inflammation, obesity, and/or regional adiposity, wherein preferably the metabolic disorder is insulin resistance, hyperinsulinemia, and/or a clinical condition associated with insulin resistance and/or hyperinsulinemia; wherein preferably said clinical condition is one or more conditions selected from impaired glucose tolerance, dyslipidemia, dysadipokinemia, subclinical inflammation, systemic inflammation, low grade systemic inflammation, obesity, and/or regional adiposity;
(ii) a metabolic disorder of an equine animal, wherein the metabolic disorder is one or more disorders selected from laminitis, vascular dysfunction, hypertension, hepatic lipidosis, atherosclerosis, hyperadrenocorticism, Pituitary Pars Intermedia Dysfunction and/or Equine Metabolic Syndrome, wherein preferably the metabolic disorder is a clinical condition/sign associated with insulin resistance and/or hyperinsulinemia, wherein said clinical condition/sign preferably is one or more conditions selected from laminitis, vascular dysfunction, hypertension, hepatic lipidosis, atherosclerosis, hyperadrenocorticism, Pituitary Pars Intermedia Dysfunction and/or Equine Metabolic Syndrome;
(iii) a metabolic disorder of a feline animal, wherein preferably the metabolic disorder is one or more selected from the group consisting of: ketoacidosis, pre-diabetes, diabetes mellitus type 1 or type 2, insulin resistance, acromegaly, diabetes with elevated IGF-1 concentration, obesity, hyperglycemia, impaired glucose tolerance, hyperinsulinemia, dyslipidemia, dysadipokinemia, subclinical inflammation, systemic inflammation, low grade systemic inflammation, hepatic lipidosis, atherosclerosis, inflammation of the pancreas, neuropathy and/or Syndrome X (metabolic syndrome) and/or loss of pancreatic beta cell function and/or wherein the remission of the metabolic disorder, preferably diabetic remission, is achieved and/or maintained;
(iv) a metabolic disorder of a canine animal, wherein preferably the metabolic disorder is one or more selected from the group consisting of: ketoacidosis, pre-diabetes, insulin dependent diabetes mellitus, insulin resistance diabetes, insulin resistance, obesity, hyperglycemia, hyperglycemia induced cataract formation, impaired glucose tolerance, hyperinsulinemia, dyslipidemia, dysadipokinemia, subclinical inflammation, systemic inflammation, low grade systemic inflammation, hepatic lipidosis, inflammation of the pancreas, metabolic disorder consequences, such as hypertension, renal dysfunction and/or musculoskeletal disorders, and/or Syndrome X (metabolic syndrome), preferably pre-diabetes, insulin dependent diabetes mellitus, insulin resistance diabetes, insulin resistance, wherein preferably the development of hyperglycemia induced cataract formation is prevented or remission is achieved and/or wherein preferably the development of metabolic disorder consequences, such as hypertension, renal dysfunction and/or musculoskeletal disorders, is prevented or progression is slowed or remission is achieved;
(v) a cardiac disease of a feline animal, wherein preferably the cardiac disease is one or more selected from the group consisting of: heart failure, heart failure due to one or more cardiomyopathies, heart failure due to hypertrophic cardiomyopathy (HCM), heart failure due to restrictive cardiomyopathy (RCM), heart failure due to dilated cardiomyopathy (DCM), heart failure due to unclassified cardiomyopathy (UCM), heart failure due to arrhythmogenic right ventricular cardiomyopathy (ARVC), hypertrophic cardiomyopathy (HCM), restrictive cardiomyopathy (RCM), dilated cardiomyopathy (DCM), unclassified cardiomyopathy (UCM), and/or arrhythmogenic right ventricular cardiomyopathy (ARVC); preferably selected from the group consisting of: heart failure due to one or more cardiomyopathies, heart failure due to hypertrophic cardiomyopathy (HCM), hypertrophic cardiomyopathy (HCM);
(vi) drying-off of a non-human mammal, preferably ruminant, improving and/or facilitating the drying-off of a non-human mammal, preferably ruminant, reducing the milk production, preferably milk production and/or secretion, in a pregnant and/or lactating non-human mammal, preferably ruminant, decreasing milk accumulation and/or engorgement in the udder, preferably udder and/or mammary gland, of a non-human mammal, preferably ruminant, decreasing the discomfort associated with udder engorgement, such as increasing the daily lying time and/or reduction of stress, of a non-human mammal, preferably ruminant, decreasing milk leakage after drying-off of a non-human mammal, preferably ruminant, decreasing the incidence of intra-mammary infections (IMI), preferably mastitis and/or metritis, in a non-human mammal, preferably ruminant;
(vii) a cardiac disease of a non-human mammal, excluding a feline, in particular a canine, wherein preferably the cardiac disease is one or more selected from the group consisting of: heart failure; congestive heart failure; asymptomatic / preclinical / occult heart failure; heart failure due to (myxomatous) mitral valve disease [(M)MVD]; congestive heart failure due to (myxomatous) mitral valve disease [(M)MVD]; asymptomatic /preclinical / occult heart failure due to (myxomatous) mitral valve disease [(M)MVD]; (myxomatous) mitral valve disease [(M)MVD]; clinically overt (myxomatous) mitral valve disease [(M)MVD]; asymptomatic / preclinical / occult (myxomatous) mitral valve disease [(M)MVD]; heart failure due to dilated cardiomyopathy (DCM); congestive heart failure due to dilated cardiomyopathy (DCM); asymptomatic / preclinical / occult heart failure due to dilated cardiomyopathy (DCM); dilated cardiomyopathy (DCM); clinically overt dilated cardiomyopathy (DCM); asymptomatic / preclinical / occult dilated cardiomyopathy (DCM); aortic stenosis (valvular, supravalvular and/or subvalvular);
(viii) hypertension in a non-human mammal, preferably a carnivore, more preferably a cat or a dog, wherein preferably the hypertension is one or more selected from the group consisting of: situational hypertension, secondary hypertension and idiopathic hypertension, wherein preferably the secondary hypertension is selected from the group consisting of hypertension associated with chronic kidney disease (CKD), diabetes, obesity, heart disease, endocrine disease, such as Cushing's disease, hyperthyroidism, acromegaly, and elevated blood pressure (BP) induced by medicaments, preferably by glucocorticoids, mineralocorticoids, erythropoiesis-stimulating agents, ephedrine and/or high dose sodium chloride;
(ix) a renal disease of a non-human mammal, preferably a carnivore, more preferably a cat or a dog, wherein preferably the renal disease is one or more selected from the group consisting of: renal dysplasia, glomerulopathy, polycystic kidney disease, amyloidosis, tubulo-nephritis / tubulointerstitial nephritis (TIN), acute kidney disease, chronic kidney disease.

The present invention further concerns a process for producing the aqueous pharmaceutical composition as described and claimed herein, comprising the steps:
(i) water is used as starting material;
(ii) adding one or more solubilizing agents to water resulting from step (i);
(iii) dissolving velagliflozin in the mixture resulting from step (ii);
(iv) optionally, dissolving the one or more preservatives in the mixture resulting from step (iii),
(v) optionally, further dissolving further excipients, such as pH modifier(s), flavor(s), sweeteners, antioxidation agents, viscosity-enhancing agents and the like, in the mixture resulting from step (iii) or optionally (iv);
(vi) optionally, filtrating the mixture resulting from step (iii), optionally step (iv) or optionally step (v); whereby, optionally, independently from each other after any of the individual process steps - be they mandatory or optional - an additional mixing step is performed.

In the course of the present invention, such process steps (i) to (vi) do not need to be carried out in the given order but can also be performed in any other meaningful order, e.g. (i), (v), (ii), (iii), (iv), (vi). It is within the knowledge of the skilled person to vary the order of process steps in order to obtain the desired process result, i.e. the aqueous pharmaceutical composition according to the present invention. For instance, if one or more viscosity-enhancing agents are added, it is preferred to heat the mixture up for complete dissolution of the one or more viscosity-enhancing agents. In turn such resulting mixture needs to be cooled down before velagliflozin (e.g., in the form of its L-proline-water cocrystal), is added in order to avoid unnecessary and unwanted degradation of the substance through such heating steps.

The present invention further concerns a kit-of-parts comprising:
(a) an aqueous pharmaceutical composition as described and claimed herein; and
(b) a package leaflet including the information that the aqueous pharmaceutical composition is to be used for the prevention and/or treatment of one or more medicinal indications in a subject in need of such prevention and/or treatment, which are selected from among the medicinal indications:
   (i) a metabolic disorder of an equine animal, wherein preferably the metabolic disorder is one or more disorders selected from insulin resistance, hyperinsulinemia, impaired glucose tolerance, dyslipidemia, dysadipokinemia, subclinical inflammation, systemic inflammation, low grade systemic inflammation, obesity, and/or regional adiposity, wherein preferably the metabolic disorder is insulin resistance, hyperinsulinemia, and/or a clinical condition associated with insulin resistance and/or hyperinsulinemia; wherein preferably said clinical condition is one or more conditions selected from impaired glucose tolerance, dyslipidemia, dysadipokinemia, subclinical inflammation, systemic inflammation, low grade systemic inflammation, obesity, and/or regional adiposity;
   (ii) a metabolic disorder of an equine animal, wherein the metabolic disorder is one or more disorders selected from laminitis, vascular dysfunction, hypertension, hepatic lipidosis, atherosclerosis, hyperadrenocorticism, Pituitary Pars Intermedia Dysfunction and/or Equine Metabolic Syndrome, wherein preferably the metabolic disorder is a clinical condition/sign associated with insulin resistance and/or hyperinsulinemia, wherein said clinical condition/sign preferably is one or more conditions selected from laminitis, vascular dysfunction, hypertension, hepatic lipidosis, atherosclerosis, hyperadrenocorticism, Pituitary Pars Intermedia Dysfunction and/or Equine Metabolic Syndrome;
   (iii) a metabolic disorder of a feline animal, wherein preferably the metabolic disorder is one or more selected from the group consisting of: ketoacidosis, pre-diabetes, diabetes mellitus type 1 or type 2, insulin resistance, acromegaly, diabetes with elevated IGF-1 concentration, obesity, hyperglycemia, impaired glucose tolerance, hyperinsulinemia, dyslipidemia, dysadipokinemia, subclinical inflammation, systemic inflammation, low grade systemic inflammation, hepatic lipidosis, atherosclerosis, inflammation of the pancreas, neuropathy and/or Syndrome X (metabolic syndrome) and/or loss of pancreatic beta cell function and/or wherein the remission of the metabolic disorder, preferably diabetic remission, is achieved and/or maintained;
   (iv) a metabolic disorder of a canine animal, wherein preferably the metabolic disorder is one or more selected from the group consisting of: ketoacidosis, pre-diabetes, insulin dependent diabetes mellitus, insulin resistance diabetes, insulin resistance, obesity, hyperglycemia, hyperglycemia induced cataract formation, impaired glucose tolerance, hyperinsulinemia, dyslipidemia, dysadipokinemia, subclinical inflammation, systemic inflammation, low grade systemic inflammation, hepatic lipidosis, inflammation of the pancreas, metabolic disorder consequences, such as hypertension, renal dysfunction and/or musculoskeletal disorders, and/or Syndrome X (metabolic syndrome), preferably pre-diabetes, insulin dependent diabetes mellitus, insulin resistance diabetes, insulin resistance, wherein preferably the development of hyperglycemia induced cataract formation is prevented or remission is achieved and/or wherein preferably the development of metabolic disorder consequences, such as hypertension, renal dysfunction and/or musculoskeletal disorders, is prevented or progression is slowed or remission is achieved.
   (v) a cardiac disease of a feline animal, wherein preferably the cardiac disease is one or more selected from the group consisting of: heart failure, heart failure due to one or more cardiomyopathies, heart failure due to hypertrophic cardiomyopathy (HCM), heart failure due to restrictive cardiomyopathy (RCM), heart failure due to dilated cardiomyopathy (DCM), heart failure due to unclassified cardiomyopathy (UCM), heart failure due to arrhythmogenic right ventricular cardiomyopathy (ARVC), hypertrophic cardiomyopathy (HCM), restrictive cardiomyopathy (RCM), dilated cardiomyopathy (DCM), unclassified cardiomyopathy (UCM), and/or arrhythmogenic right ventricular cardiomyopathy (ARVC); preferably selected from the group consisting of: heart failure due to one or more cardiomyopathies, heart failure due to hypertrophic cardiomyopathy (HCM), hypertrophic cardiomyopathy (HCM);
   (vi) drying-off of a non-human mammal, preferably ruminant, improving and/or facilitating the drying-off of a non-human mammal, preferably ruminant, reducing the milk production, preferably milk production and/or secretion, in a pregnant and/or lactating non-human mammal, preferably ruminant, decreasing milk accumulation and/or engorgement in the udder, preferably udder and/or mammary gland, of a non-human mammal, preferably ruminant, decreasing the discomfort associated with udder engorgement, such as increasing the daily lying time and/or reduction of stress, of a non-human mammal, preferably ruminant, decreasing milk leakage after drying-off of a non-human mammal, preferably ruminant, decreasing the incidence of intra-mammary infections (IMI), preferably mastitis and/or metritis, in a non-human mammal, preferably ruminant
   (vii) a cardiac disease of a non-human mammal, excluding a feline, in particular a canine, wherein preferably the cardiac disease is one or more selected from the group consisting of: heart failure; congestive heart failure; asymptomatic / preclinical / occult heart failure; heart failure due to (myxomatous) mitral valve disease [(M)MVD]; congestive heart failure due to (myxomatous) mitral valve disease [(M)MVD]; asymptomatic /preclinical / occult heart failure due to (myxomatous) mitral valve disease [(M)MVD]; (myxomatous) mitral valve disease [(M)MVD]; clinically overt (myxomatous) mitral valve disease [(M)MVD]; asymptomatic / preclinical / occult (myxomatous) mitral valve disease [(M)MVD]; heart failure due to dilated cardiomyopathy (DCM); congestive heart failure due to dilated cardiomyopathy (DCM); asymptomatic / preclinical / occult heart failure due to dilated cardiomyopathy (DCM); dilated cardiomyopathy (DCM); clinically overt dilated cardiomyopathy (DCM); asymptomatic / preclinical / occult dilated cardiomyopathy (DCM); aortic stenosis (valvular, supravalvular and/or subvalvular);
   (viii) hypertension in a non-human mammal, preferably a carnivore, more preferably a cat or a dog, wherein preferably the hypertension is one or more selected from the group consisting of: situational hypertension, secondary hypertension and idiopathic hypertension, wherein preferably the secondary hypertension is selected from the group consisting of hypertension associated with chronic kidney disease (CKD), diabetes, obesity, heart disease, endocrine disease, such as Cushing's disease, hyperthyroidism, acromegaly, and elevated blood pressure (BP) induced by medicaments, preferably by glucocorticoids, mineralocorticoids, erythropoiesis-stimulating agents, ephedrine and/or high dose sodium chloride;
   (ix) a renal disease of a non-human mammal, preferably a carnivore, more preferably a cat or a dog, wherein preferably the renal disease is one or more selected from the group consisting of: renal dysplasia, glomerulopathy, polycystic kidney disease, amyloidosis, tubulo-nephritis / tubulointerstitial nephritis (TIN), acute kidney disease, chronic kidney disease.

The advantages of the aqueous pharmaceutical compositions according to the present invention are as follows:
- They are suitable for, preferably direct, administration to a subject, preferably without further mandatory processing and/or purification steps. Preferably, they are therefore sterile and comply with GMP manufacturing conditions as well as GCP compliant clinical protocols;
- They are stable against undesired contamination by / growth of microorganisms;
- They are preferably true solutions, emulsions or suspensions, more preferably true solutions, in an aqueous based system with a minimum amount of organic solvents (if any) ensuring biocompatibility and reduction in costs.

### DETAILED DESCRIPTION OF THE INVENTION

Before the embodiments of the present invention are described in further details it shall be noted that as used herein and in the appended claims, the singular forms "a", "an", and "the" include plural reference unless the context clearly dictates otherwise.

Unless defined otherwise, all technical and scientific terms used herein have the same meanings as commonly understood by one of ordinary skill in the art to which this invention belongs. All given ranges and values may vary by 1 to 5 % unless indicated otherwise or known otherwise by the person skilled in the art, therefore, the term "about" was usually omitted from the description and claims. Although any methods and materials similar to those described herein can be used in the practice or testing of the present invention, the preferred methods, devices, and materials are now described.
SGLT-2 inhibitors are known in the art and include Dapagliflozin, Canagliflozin, Empagliflozin, Luseogliflozin, Tofogliflozin, Ipragliflozin, Ertugliflozin, Atigliflozin, Remogliflozin, Remogliflozin etabonate, and glucopyranosyl-substituted benzene derivatives, for example as described in WO 01/27128, WO 03/099836, WO 2005/092877, WO 2006/034489, WO 2006/064033, WO 2006/117359, WO 2006/117360, WO 2007/025943, WO 2007/028814, WO 2007/031548, WO 2007/093610, WO 2007/128749, WO 2008/049923, WO 2008/055870, WO 2008/055940, WO 2009/022020 or WO 2009/022008. The SGLT-2 inhibitor comprised in the aqueous pharmaceutical composition of the invention is
Velagliflozin, represented by formula (2):

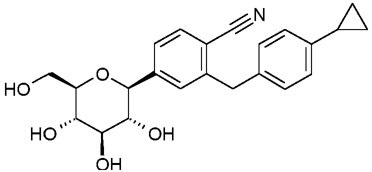

The term "velagliflozin" as employed herein refers to velagliflozin of the above structure as well as pharmaceutically acceptable forms thereof, including hydrates and solvates thereof, and crystalline forms thereof. The compound, methods of its synthesis and co-crystals thereof are described in WO 2007/128749, WO 2014/016381 and WO 2019/121509 for example.

In the course of the present invention 1-cyano-2-(4-cyclopropyl-benzyl)-4-(β-D-glucopyranos-1-yl)-benzene (velagliflozin) is also referred to as "the substance" and is herewith understood to also comprise co-crystal 1-cyano-2-(4-cyclopropyl-benzyl)-4-(β-D-glucopyranos-1-yl)-benzene - L-proline as well as the 1:1:1-co-crystal 1-cyano-2-(4-cyclopropyl-benzyl)-4-(β-D-glucopyranos-1-yl)-benzene - L-proline - water (as disclosed in WO 2014/016381). Generally, in the case of disclosed and claimed concentrations (% w/v) and amounts (g, mg) the concentration or amount always refers to 1-cyano-2-(4-cyclopropyl-benzyl)-4-(β-D-glucopyranos-1-yl)-benzene as such, i.e. excluding L-proline and crystal water, unless otherwise explicitly stated -even though in practice (and in the example section) 1:1:1 co-crystal 1-cyano-2-(4-cyclopropyl-benzyl)-4-(β-D-glucopyranos-1-yl)-benzene - L-proline - water is actually added/used.

In the course of the present invention the term "for, preferably direct, administration to a subject" in connection with "aqueous pharmaceutical composition" means that such aqueous pharmaceutical compositions can be directly administrated to a subject, preferably without further mandatory processing and/or purification steps, and explicitly excludes (mixtures of) organic solvents that are solely mentioned in the context of producing crystalline complexes of SGLT-2 inhibitors. Preferably, such "aqueous pharmaceutical composition" that are "for direct administration to a subject" are therefore sterile and/or comply with GMP manufacturing conditions as well as GCP compliant clinical protocols.

In one aspect, the present invention relates to an aqueous pharmaceutical composition as described and/or claimed herein, wherein the aqueous pharmaceutical composition is for, preferably direct, administration to a subject, preferably without further mandatory processing and/or purification steps, more preferably an animal, even more preferably a mammal, in particular a horse, cat, dog or cow; wherein preferably the aqueous pharmaceutical composition is sterile.

In another aspect, the present invention relates to an aqueous pharmaceutical composition as described and/or claimed herein, wherein the aqueous pharmaceutical composition is a solution, an emulsion or a suspension, preferably a solution, an emulsion or a suspension with an NTU value of equal to or less than 10.0, more preferably equal to or less than 7.0, even more preferably equal to or less than 3.0, and most preferably a solution, in particular a solution with an NTU value of equal to or less than 3.0.

In the course of the present invention the term "NTU" refers to Nephelometric Turbidity Units and to an opalescent value as defined and described in European Pharmacopoeia 8th edition (Ph. Eur. 8, Chapter 2.2.1. "Clarity and degree of opalescence of liquids").

The present disclosure relates to an aqueous pharmaceutical composition which contains one or more organic solvents that are independently from each other characterized by a negative log₁₀P value, preferably a negative decadic logarithmic partition coefficient (P) in an n-octanol/water system according to formula (II): 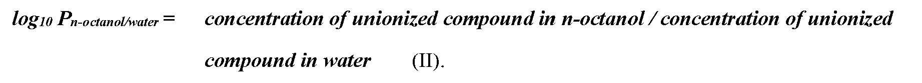 log10 Pn-octanol/water = concentration of unionized compound in n-octanol / concentration of unionized compound in water (II).

In a further aspect of the disclosure, such aqueous pharmaceutical composition as a whole is characterized by a negative LogP-Parameter, preferably a negative LogP-Parameter of equal to or higher than -2.0 (i.e.-2.0 ≤ LogP-Parameter < 0). For the avoidance of doubt, the LogP-Parameter is defined as in Eq. 4 of Example 1 and is not identical with and should not be mistaken for the (negative) log₁₀P value as given for a(n) organic solvent(s).

In yet another aspect of the disclosure, the one or more organic solvents are selected from the group consisting of: ethanol (log₁₀P: -0.16), propane-1,2,3-triol (glycerol; log₁₀P: -1.84), pyrrolidone (log₁₀P: -0.58), methylpyrrolidone (log₁₀P: -0.356), N,N-dimethylacetamide (log₁₀P: -0.583) and/or N,N-dimethylformamide (log₁₀P: -0.632). The log₁₀P values were taken from http://www.chemicalize.org/.

The present invention relates to an aqueous pharmaceutical composition as described and/or claimed herein, wherein such composition is substantially free of organic solvents, preferably does not contain any organic solvents. In case no organic solvent is present, the solvent of the aqueous pharmaceutical composition is 100% water, preferably in the form of aqueous buffer.

In yet another aspect, the present invention relates to an aqueous pharmaceutical composition as described and/or claimed herein, wherein such aqueous pharmaceutical composition comprises water in the form of aqueous buffer, such as citric acid buffer, phosphate buffer, ammonium sulphate buffer, ammonium acetate buffer, sodium acetate buffer and the like, preferably aqueous buffer with pH 4.5, more preferably citric acid buffer with pH 4.5.

In yet another aspect, the present invention relates to an aqueous pharmaceutical composition as described and/or claimed herein, wherein such aqueous pharmaceutical composition contains from 30 to 100 g/100 mL (30 to 100 % w/v), preferably from 30 to 95 g/100 mL (30 to 95 % w/v), more preferably from 30 to 90 g/100 mL (30 to 90 % w/v), more preferably from 30 to 85 g/100 mL (30 to 85 % w/v), even more preferably from 30 to 80 g/100 mL (30 to 80 % w/v), most preferably from 50 to 80 g/100 mL (50 to 80 % w/v) water, more preferably in the form of aqueous buffer, such as citric acid buffer, even more preferably aqueous buffer with pH 4.5, most preferably citric acid buffer with pH 4.5.

In yet another aspect, the present invention relates to an aqueous pharmaceutical composition as described and/or claimed herein, wherein such aqueous pharmaceutical composition has a (measured) pH value from 2 to 7, preferably from 3 to 7, more preferably from 3.0 to 6.5, even more preferably from 4.0 to 6.5, even more preferably from 4.0 to 5.0, and most preferably of 4.5. For the avoidance of doubt, the term "measured pH value" refers to the pH value actually measured for the whole aqueous pharmaceutical composition according to the present invention, i.e. comprising both organic solvent(s) phase, if any, and aqueous phase.

In yet another aspect, the present invention relates to an aqueous pharmaceutical composition as described and/or claimed herein, wherein such aqueous pharmaceutical composition additionally comprises one or more preservatives, preferably selected from the group consisting of: sorbic acid or salts thereof, preferably sodium sorbate, potassium sorbate, calcium sorbate; benzoic acid or salts thereof, preferably sodium benzoate; benzalkonium chloride; benzethonium chloride; benzyl alcohol, cetylpyridinium chloride; sodium metabisulfite; sodium acetate; parabens and salts thereof, preferably methylparaben, ethylparaben, propylparaben, butylparaben, butylparaben sodium; most preferably benzoic acid and/or salts thereof, such as sodium benzoate.

In yet another aspect, the present invention relates to an aqueous pharmaceutical composition as described and/or claimed herein, wherein such aqueous pharmaceutical composition additionally comprises one or more antioxidation agents, preferably selected from the group consisting of: ascorbic acid or pharmaceutically acceptable salts thereof, particularly sodium ascorbate; citric acid (anhydrous and/or monohydrate) or pharmaceutically acceptable salts thereof, more preferably sodium citrate; erythorbic acid; fumaric acid; malic acid; monothioglycerol; phosphoric acid; sodium metabisulfite; potassium metabisulfite; propionic acid; sodium bisulfite; sodium sulfite; resveratrol; butylhydroxyanisol; butylhydroxytoluene; gallate derivatives, particularly propylgallate, octylgallate; Vitamin E or pharmaceutically acceptable salts thereof; ascorbyl palmitate; edetic acid or pharmaceutically acceptable salts thereof; most preferably sodium metabisulfite.

In yet another aspect, the present invention relates to an aqueous pharmaceutical composition as described and/or claimed herein, wherein the one or more solubilizing agents are selected from the group consisting of: surfactants, anionic surfactants, non-ionic surfactants, hydrogenated castor oils, polyoxyethylene-polyoxypropylene block copolymers, polyethylene glycols, propylene glycol derivatives, polyvinylpyrrolidones, more preferably selected from the group consisting of: sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate, Kollidon 12 (povidone), wherein preferably the total amount of the one or more solubilizing agents is from 1 to 50 g/100 mL (1 - 50 % w/v), more preferably from 1 to 45 g/100 mL (1 - 45 % w/v), even more preferably from 1 to 40 g/100 mL (1 - 40 % w/v), even more preferably from 1 to 35 g/100 mL (1 - 35 % w/v), even more preferably from 1 to 30 g/100 mL (1 - 30 % w/v), even more preferably from 1 to 25 g/100 mL (1 - 25 % w/v), most preferably from 5 to 25 g/100 mL (5 - 25 % w/v).

In yet another aspect, the present invention relates to an aqueous pharmaceutical composition as described and/or claimed herein, wherein two or more solubilizing agents are contained in the aqueous pharmaceutical composition.

In yet another aspect, the present invention relates to an aqueous pharmaceutical composition as described and/or claimed herein, wherein two solubilizing agents are contained in such aqueous pharmaceutical composition, wherein preferably the amount of the first solubilizing agent and the amount of second solubilizing agent are independently from each other selected from 1 to 50 g/100 mL (1 - 50 % w/v), more preferably from 1 to 45 g/100 mL (1 - 45 % w/v), even more preferably from 1 to 40 g/100 mL (1 - 40 % w/v), even more preferably from 1 to 35 g/100 mL (1 - 35 % w/v), even more preferably from 1 to 30 g/100 mL (1 - 30 % w/v), even more preferably from 1 to 25 g/100 mL (1 - 25 % w/v), most preferably from 5 to 25 g/100 mL (5 - 25 % w/v), wherein more preferably these two solubilizing agents are independently from each other selected from the group consisting of: sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate, Kollidon 12 (povidone), wherein even more preferably, such two solubilizing agents are Kollidon 12 (povidone) as well as PEG 200, PEG 300, or PEG 400, most preferably Kollidon 12 (povidone) and PEG 300.

In yet another aspect, the present invention relates to an aqueous pharmaceutical composition as described and/or claimed herein, wherein such aqueous pharmaceutical composition additionally comprises one or more viscosity-enhancing agents, preferably selected from the group consisting of: inorganic gel forming agents, organic gel forming agents, cellulose derivatives, more preferably selected from the group consisting of: hydroxyl ethyl cellulose, hydroxyl propyl methyl cellulose, silicon dioxide.

In yet another aspect, the present invention relates to an aqueous pharmaceutical composition as described and/or claimed herein, wherein such aqueous pharmaceutical composition additionally comprises one or more flavours and/or sweeteners, preferably selected from the group consisting of: honey flavor, lime/salvia flavor, jasmine flavor, lavender flavor, peppermint flavor, raspberry flavor, lemon flavor, herbs flavor, meat flavor, vanillin/vanilla flavor, saccharine, aspartame, sorbitol, xylitol.

In yet another aspect, the present invention relates to an aqueous pharmaceutical composition as described and/or claimed herein, wherein such aqueous pharmaceutical composition is for oral and/or parenteral administration, preferably oral administration.

In yet another aspect, the present invention relates to an aqueous pharmaceutical composition as described and/or claimed herein, comprising, preferably consisting of:
(i) only velagliflozin as single SGLT-2 inhibitor;
(ii) optionally, but preferred, one or more preservatives; preferably selected from the group consisting of: sorbic acid or salts thereof, preferably sodium sorbate, potassium sorbate, calcium sorbate; benzoic acid or salts thereof, preferably sodium benzoate; benzalkonium chloride; benzethonium chloride; benzyl alcohol, cetylpyridinium chloride; sodium metabisulfite; sodium acetate; parabens and salts thereof, preferably methylparaben, ethylparaben, propylparaben, butylparaben, butylparaben sodium; or combinations thereof; most preferably benzoic acid and/or salts thereof, such as sodium benzoate;
(iii) a composition which is substantially free of organic solvents, preferably does not contain any organic solvents;
(iv) optionally, one or more flavors and/or sweeteners, preferably selected from the group consisting of: honey flavor, lime/salvia flavor, jasmine flavor, lavender flavor, peppermint flavor, raspberry flavor, lemon flavor, herbs flavor, meat flavor, vanillin/vanilla flavor, saccharine, aspartame, sorbitol, xylitol;
(v) water, preferably in the form of aqueous buffer, more preferably citric acid buffer, even more preferably aqueous buffer with pH 4.5, most preferably citric acid buffer with pH 4.5;
(vi) one or more solubilizing agents, preferably selected from the group consisting of: surfactants, anionic surfactants, non-ionic surfactants, hydrogenated castor oils, polyoxyethylene-polyoxypropylene block copolymers, polyethylene glycols, propylene glycol derivatives, polyvinylpyrrolidones, more preferably selected from the group consisting of: sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate, Kollidon 12 (povidone);
(vii) optionally, one or more viscosity-enhancing agents, preferably selected from the group consisting of: inorganic gel forming agents, organic gel forming agents, cellulose derivatives, more preferably selected from the group consisting of: hydroxyl ethyl cellulose, hydroxyl propyl methyl cellulose, silicon dioxide;
(viii) optionally, one or more antioxidation agents, preferably selected from the group consisting of: ascorbic acid or pharmaceutically acceptable salts thereof, particularly sodium ascorbate; citric acid (anhydrous and/or monohydrate) or pharmaceutically acceptable salts thereof, more preferably sodium citrate; erythorbic acid; fumaric acid; malic acid; monothioglycerol; phosphoric acid; sodium metabisulfite; potassium metabisulfite; propionic acid; sodium bisulfite; sodium sulfite; resveratrol; butylhydroxyanisol; butylhydroxytoluene; gallate derivatives, particularly propylgallate, octylgallate; Vitamin E or pharmaceutically acceptable salts thereof; ascorbyl palmitate; edetic acid or pharmaceutically acceptable salts thereof; most preferably sodium metabisulfite.

In yet another aspect, the present invention relates to an aqueous pharmaceutical composition as described and/or claimed herein, comprising, preferably consisting of:
(i) 0.5 - 20.0 g/100 mL (0.5 - 20.0 % w/v), preferably 0.5 - 15.0 g/100 mL (0.5 - 15.0 % w/v), more preferably 1.0 -1.5 g/100 mL (1.0 - 1.5 % w/v) velagliflozin as single SGLT-2 inhibitor;
(ii) 0 - 3.0 g/100 mL (0 - 3.0 % w/v), preferably 0.05 - 3.0 g/100 mL (0.05 - 3.0 % w/v), more preferably 0.05 - 2.0 g/100 mL (0.05 - 2.0 % w/v) one or more preservatives; even more preferably selected from the group consisting of: sorbic acid or salts thereof, preferably sodium sorbate, potassium sorbate, calcium sorbate; benzoic acid or salts thereof, preferably sodium benzoate; benzalkonium chloride; benzethonium chloride; benzyl alcohol, cetylpyridinium chloride; sodium metabisulfite; sodium acetate; parabens and salts thereof, preferably methylparaben, ethylparaben, propylparaben, butylparaben, butylparaben sodium; or combinations thereof; most preferably benzoic acid and/or salts thereof, such as sodium benzoate;
(iii) a composition which is substantially free of organic solvents, preferably does not contain any organic solvents;
(iv) 0 - 40 g/100 mL (0 - 40 % w/v), preferably 0 - 30 g/100 mL (0 - 30 % w/v), more preferably 0 - 2 g/100 mL (0 - 2 % w/v) one or more flavors and/or sweeteners, more preferably selected from the group consisting of: honey flavor, lime/salvia flavor, jasmine flavor, lavender flavor, peppermint flavor, raspberry flavor, lemon flavor, herbs flavor, meat flavor, vanillin/vanilla flavor, saccharine, aspartame, sorbitol, xylitol;
(v) 30 to 100 g/100 mL (30 to 100 % w/v), preferably from 30 to 95 g/100 mL (30 to 95 % w/v), more preferably from 30 to 90 g/100 mL (30 to 90 % w/v), more preferably from 30 to 85 g/100 mL (30 to 85 % w/v), even more preferably from 30 to 80 g/100 mL (30 to 80 % w/v), most preferably from 50 to 80 g/100 mL (50 to 80 % w/v) water, preferably in the form of aqueous buffer, more preferably citric acid buffer, even more preferably aqueous buffer with pH 4.5, most preferably citric acid buffer with pH 4.5;
(vi) 1 to 50 g/100 mL (1 - 50 % w/v), preferably from 1 to 45 g/100 mL (1 - 45 % w/v), even more preferably from 1 to 40 g/100 mL (1 - 40 % w/v), even more preferably from 1 to 35 g/100 mL (1 - 35 % w/v), even more preferably from 1 to 30 g/100 mL (1 - 30 % w/v), even more preferably from 1 to 25 g/100 mL (1 - 25 % w/v), most preferably from 5 to 25 g/100 mL (5 - 25 % w/v) one or more solubilizing agents, preferably selected from the group consisting of: surfactants, anionic surfactants, non-ionic surfactants, hydrogenated castor oils, polyoxyethylene-polyoxypropylene block copolymers, polyethylene glycols, propylene glycol derivatives, polyvinylpyrrolidones, more preferably selected from the group consisting of: sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate, Kollidon 12 (povidone);
(vii) 0 - 40 g/100 mL (0 - 40 % w/v), preferably 10 - 30 g/100 mL (10 - 30 % w/v), one or more viscosity-enhancing agents, preferably selected from the group consisting of: inorganic gel forming agents, organic gel forming agents, cellulose derivatives, more preferably selected from the group consisting of: hydroxyl ethyl cellulose, hydroxyl propyl methyl cellulose, silicon dioxide;
(viii) 0 - 1.0 g/100 mL (0 - 1.0 % w/v), preferably 0 - 0.5 g/100 mL (0 - 0.5 % w/v), more preferably 0.1 - 0.3 g/100 mL (0.1 - 0.3 % w/v), one or more antioxidation agents, preferably selected from the group consisting of: ascorbic acid or pharmaceutically acceptable salts thereof, particularly sodium ascorbate; citric acid (anhydrous and/or monohydrate) or pharmaceutically acceptable salts thereof, more preferably sodium citrate; erythorbic acid; fumaric acid; malic acid; monothioglycerol; phosphoric acid; sodium metabisulfite; potassium metabisulfite; propionic acid; sodium bisulfite; sodium sulfite; resveratrol; butylhydroxyanisol; butylhydroxytoluene; gallate derivatives, particularly propylgallate, octylgallate; Vitamin E or pharmaceutically acceptable salts thereof; ascorbyl palmitate; edetic acid or pharmaceutically acceptable salts thereof; most preferably sodium metabisulfite.

In yet another aspect, the present invention relates to an aqueous pharmaceutical composition as described and/or claimed herein, comprising, preferably consisting of: velagliflozin; sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate and/or Kollidon 12 (povidone); sodium benzoate; optionally, Na-metabisulfite; water.

In yet another aspect, the present invention relates to an aqueous pharmaceutical composition as described and/or claimed herein, comprising, preferably consisting of: velagliflozin; citric acid monohydrate; NaOH; sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate and/or Kollidon 12 (povidone); sodium benzoate; optionally, Na-metabisulfite; water.

In yet another aspect, the present invention relates to an aqueous pharmaceutical composition as described and/or claimed herein, comprising, preferably consisting of: velagliflozin; citric acid monohydrate; NaOH; sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate and/or Kollidon 12 (povidone); sodium benzoate; optionally, Na-metabisulfite; sorbitol and/or xylitol; honey flavor and/or vanillin and/or meat flavor; water.

In yet another aspect, the present invention relates to an aqueous pharmaceutical composition as described and/or claimed herein, selected from Compositions 5 to 10. The Compositions 1 to 4 are reference compositions.

| Ingredient | Composition 1 [mg/mL] | Composition 2 [mg/mL] | Composition 3 [mg/mL] | Composition 4 [mg/mL] |
|---|---|---|---|---|
| Velagliflozin | 15.0 | 15.0 | 15.0 | 15.0 |
| Citric Acid Monohydrate | 10.1 | 9.2 | 9.2 | 9.8 |
| NaOH 1 M | 60.0 | 53.6 | 54.4 | 46.4 |
| Kollidon 12 | 75.0 | 75.0 | 75.0 | - |
| PEG 300 | - | - | - | 150.0 |
| Sodium benzoate | 2.0 | 2.0 | 2.0 | 2.0 |
| Na-metabisulfite | 2.0 | 2.0 | 2.0 | - |
| Ethanol, absolut | 80.0 | 80.0 | 80.0 | 80.0 |
| Sorbitol | 170.0 | 170.0 | 170.0 | 170.0 |
| Xylitol | 100.0 | 100.0 | - | 91.8 |
| Honey Flavor | 1.5 | - | - | 1.5 |
| Vanillin | - | 15.0 | - | - |
| Meat Flavor | - | - | 1.0 | - |
| Water, purified | 584.3 | 581.2 | 659.4 | 536.5 |

| Ingredient | Composition 5 [mg/mL] | Composition 6 [mg/mL] | Composition 7 [mg/mL] | Composition 8 [mg/mL] |
|---|---|---|---|---|
| Velagliflozin | 15.0 | 15.0 | 15.0 | 15.0 |
| Citric Acid Monohydrate | 9.8 | 9.8 | 9.8 | 9.8 |
| NaOH 1 M | 46.4 | 46.4 | 46.4 | 46.4 |
| Kollidon 12 | - | 100.0 | 50.0 | - |
| PEG 300 | 150.0 | - | 100.0 | - |
| Sodium dodecyl sulphate (SDS; Na-Lauryl-Sulfate) | - | - | - | 50.0 |
| Sodium benzoate | 2.0 | 2.0 | 2.0 | 2.0 |
| Na-metabisulfite | - | 2.0 | 2.0 | - |
| Sorbitol | 170.0 | 170.0 | 170.0 | 170.0 |
| Honey Flavor | 1.5 | 1.5 | 1.5 | 1.5 |
| Water, purified | ad. 1100 ml | ad. 1100 ml | ad. 1100 ml | Ad. 1100 ml |

| Ingredient | Composition 9 [mg/mL] | Composition 10 [mg/mL] | Composition 11 [mg/mL] | Composition 12 [mg/mL] |
|---|---|---|---|---|
| Velagliflozin | 15.0 | 15.0 | 15.0 | 15.0 |
| Citric Acid Monohydrate | 9.8 | 9.8 | 9.8 | 9.8 |
| NaOH 1 M | 46.4 | 46.4 | 46.4 | 46.4 |
| Cremophor RH40 | 250.0 | - | - | - |
| Polysorbate 80 | - | 250.0 | - | - |
| Poloxamer 188 | - | - | 50.0 | - |
| PEG 400 | - | - | - | 200.0 |
| Sodium benzoate | 2.0 | 2.0 | 2.0 | 2.0 |
| Sorbitol | 170.0 | 170.0 | 170.0 | 170.0 |
| Honey Flavor | 1.5 | 1.5 | 1.5 | 1.5 |
| Water, purified | ad. 1100 ml | ad. 1100 ml | ad. 1100 ml | Ad. 1100 ml |

### EXAMPLES

The following examples serve to further illustrate the present invention; but the same should not be construed as a limitation of the scope of the invention disclosed herein.

### Example 1

The testing criteria applied are those for evaluation of the clarity of a liquid (formulation) comprising 1-cyano-2-(4-cyclopropyl-benzyl)-4-(β-D-glucopyranos-1-yl)-benzene (velagliflozin) according to Pharm. Eur. 8. Concerning the Ph. Eur. 8, Chapter 2.2.1. "Clarity and degree of opalescence of liquids", a liquid is considered clear if its opalescence is not more pronounced than that of reference suspension I having an opalescent value of 3 NTU (Table 1).

**Table 1: Measurements of reference suspensions I-IV according Pharm. Eur. 8, Chapter 2.2.1**

| **Formazin suspensions** | **Opalescent values (NTU)** |
|---|---|
| Reference suspension I | 3 |
| Reference suspension II | 6 |
| Reference suspension III | 18 |
| Reference suspension IV | 30 |
| Standard of opalescence | 60 |
| Primary opalescent suspension | 4000 |

In the following, exemplary pharmaceutical compositions of solvents which were mixed with 1-cyano-2-(4-cyclopropyl-benzyl)-4-(β-D-glucopyranos-1-yl)-benzene (velagliflozin; the substance) according to the present invention are given in detail. The turbidity was measured by using a Hach Lange 2100 N IS apparatus.

In order to quantify the solvent characteristics regarding their suitability to form a physical stable solution with the substance, a LogP-Parameter was introduced (Eq. 4). The LogP-Parameter describes the hydrophilic/hydrophobic nature of solvent mixture containing organic and aqueous solvents and is calculated as follows: ${Mo}_{i} = \frac{{mo}_{i}}{{\sum }_{i = 1}^{n} {mo}_{i}}$ (Eq. 1) $LogPo = {\sum }_{i = 1}^{n} \left({Mo}_{i}⋅{LogP}_{i}\right)$ (Eq. 2) ${X}_{o} = 1 - \frac{{a}_{w}}{{a}_{sol}}$ (Eq. 3) $LogP Parameter = \frac{LogPo}{{X}_{o}}$ (Eq. 4)
- *moᵢ* [mol/g]:: molecular amount of an organic solvent in the organic phase of a solvent mixture
- *Moᵢ* [-]:: molecular fraction of organic solvent *moᵢ* in the organic phase of a solvent mixture
- LogPᵢ [-]:: log₁₀ P_{n-octanol/water} = concentration of unionized compound in n-octanol / concentration of unionized compound in water of an organic solvent
- LogPo [-]:: auxiliary parameter of the organic phase of a solvent mixture
- *a_{w}* [g]:: mass of water or aqueous buffer in a solvent mixture
- *aₛₒₗ* [g]:: mass of solvent mixture
- *Xₒ* [-]:: mass fraction of organic phase in a solvent mixture

As described in WO 2017/032799 instability phenomena are expected for LogP-Parameters < -2.0.

### Exemplary calculation

For ethanol the log₁₀P value (= LogPᵢ) is given as -0.16 and the molecular weight as 46.07 g/mol. The solvent mixture of Composition 1 consists of 80.0 mg/mL ethanol and 644.3 mg/mL H₂O (sum of water purified and aqueous buffer), which corresponds to an organic phase of 11.0% (m/m).

Because ethanol is the only organic solvent, the amount of ethanol in the organic phase is 100% (m/m) or 100 g/100g which corresponds to 0.0217 mol/g (*=moₑₜₕₐₙₒₗ*). Therefore, *Moₑₜₕₐₙₒₗ* is 1 (Eq. 1) and LogPo is calculated as -0.16 (Eq. 2). The mass fraction of the organic phase is 11.0% (m/m) or according to Eq. 3 *Xₒ* = 0.11. Following Eq. 4 a LogP Parameter of -1.45 is calculated for the solvent mixture of Composition 1.

### Example 2

In the following Table 2, exemplary pharmaceutical compositions according to the present invention are given in detail (API: active pharmaceutical ingredient).

**Table 2: Exemplary pharmaceutical compositions according to the present invention**

| Ingredient | Concentration [mg/mL] | Function |
|---|---|---|
| velagliflozin) | 5 - 200; preferably 5 - 150, more preferably 10 -15 | API |
| Kollidon 12 | 1 - 500, preferably 1 - 450, more preferably 1 - 400, even more preferably 1- 350, even more preferably 1 - 300; even more preferably 1 - 250, most preferably 50 - 250 | Solubilizer |
| Sodium Benzoate | 0 - 30; preferably 0.5 - 30, more preferably 0.5 - 20 | Preservative |
| Ethanol, abs. | 0 - | Organic Solvent |
| Flavor | 0 - 30; preferably 0 - 20 | Flavor |
| Sodium metabisulfite | 0 - 10; preferably 1 - 3 | Antioxidant |
| Sweetener (e.g., Sorbitol, Xylitol) | 0 - 400; preferably 100 - 300 | Sweetener/Viscosity Enhancer |
| Aqueous buffer (e.g., citric acid buffer pH 4.5) | 300 - 1000, 300 - 950, 300 - 900, 300 - 850; 300 - 800, preferably 500 - 800 | Water (pH adjusted) |

The production procedure of an exemplary pharmaceutical composition according to the present invention for a single small-scale batch (1000 mL) in form of a general instruction is as follows:
Prepare buffer solution

Weigh aqueous buffer solution in a vessel.

Weigh solubilizer components and add to buffer solution under stirring.

Weigh preservative and add to the solution under stirring (if any).

Weigh antioxidant and add to the solution under stirring.

Weigh sweetener and/or viscosity-enhancer and add to the solution under stirring.

Weigh the at least one SGLT-2 inhibitor, preferably velagliflozin, and add in portions to the solution.

Weigh flavor and add to the solution under stirring

Stir until fully dissolved.

Filtration of the solution.

### Example 3

Formulation samples were produced with compositions listed in the following Table 3 (API: active pharmaceutical ingredient).

**Table 3: Exemplary pharmaceutical compositions according to the present invention**

| Ingredient | Concentration [mg/mL] | Function |
|---|---|---|
| SGLT-2 inhibitor (e.g. velagliflozin) | 5 - 200; preferably 5 - 150, more preferably 10 -15 | API |
| PEG (e.g., PEG200, PEG300, PEG400) | 1 - 500, preferably 1 - 450, more preferably 1 - 400, even more preferably 1- 350, even more preferably 1 - 300; even more preferably 1 - 250, most preferably 50 - 250 | Solubilizer |
| Sodium Benzoate | 0 - 30, preferably 0.5 - 30; more preferably 0.5 - 20 | Preservative |
| Ethanol, abs. | 0 | Organic Solvent |
| | 1 | |
| Flavor | 0 - 30; preferably 0 - 20 | Flavor |
| Sweetener (e.g., Sorbitol, Xylitol) | 0 - 400; preferably 100 - 300 | Sweetener/Viscosity Enhancer |
| Aqueous buffer (e.g., citric acid buffer pH 4.5) | 300 - 1000, 300 - 950, 300 - 900, 300 - 850; 300 - 800, preferably 500 - 800 | Water (pH adjusted) |

The following procedure was used to prepare the samples:
1. Weigh entire amount of aqueous buffer into vessel
2. Weigh entire amount of PEG into a beaker and add slowly to stirred solution.
4. Weigh preservative and add to the solution under stirring (if any).
5. Weigh entire amount of the at least one SGLT-2 inhibitor, preferably velagliflozin, into a beaker and add in portions to stirred solution. Stir until fully dissolved.
6. Weigh entire amount of flavour into a beaker and add to stirred solution. Stir until fully dissolved.
7. Weigh entire amount of sweetener and/or viscosity enhancer and add to the solution under stirring.
8. Stir until fully dissolved.
9. Use an 8 µm filter to filtrate the solution

### Example 4 (Reference)

Formulation samples were produced with compositions listed in the following Table 4.

**Table 4: Exemplary pharmaceutical compositions according to the present invention**

| Ingredient | Composition 1 [mg/mL] | Composition 2 [mg/mL] | Composition 3 [mg/mL] | Composition 4 [mg/mL] |
|---|---|---|---|---|
| Velagliflozin | 15.0 | 15.0 | 15.0 | 15.0 |
| Citric Acid Monohydrate | 10.1 | 9.2 | 9.2 | 9.8 |
| NaOH 1 M | 60.0 | 53.6 | 54.4 | 46.4 |
| Kollidon 12 | 75.0 | 75.0 | 75.0 | - |
| PEG 300 | - | - | - | 150.0 |
| Sodium benzoate | 2.0 | 2.0 | 2.0 | 2.0 |
| Na metabisulfite | 2.0 | 2.0 | 2.0 | - |
| Ethanol, absolut | 80.0 | 80.0 | 80.0 | 80.0 |
| Sorbitol | 170.0 | 170.0 | 170.0 | 170.0 |
| Xylitol | 100.0 | 100.0 | - | 91.8 |
| Honey Flavor | 1.5 | - | - | 1.5 |
| Vanillin | - | 15.0 | - | - |
| Meat Flavor | - | - | 1.0 | - |
| Water, purified | 584.3 | 581.2 | 659.4 | 536.5 |

The following procedure was used to prepare the samples:
Weigh entire amount of water into vessel

Weigh entire amounts of NaOH 1N and citric acid monohydrate into a beaker and add to stirred water. Stir until fully dissolved.

Weigh entire amount of solubilizers (Kollidon 12 or PEG 300) into a beaker and add slowly to stirred solution.

Weigh entire amount of ethanol, abs. into a beaker and add to stirred solution. Stir until fully mixed.

Weigh antioxidant (if needed) and preservative and add to the solution under stirring.

Weigh entire amount of sweetener and/or viscosity enhancer and add to the solution under stirring.

Weigh entire amount of flavour into a beaker and add to stirred solution. Stir until fully dissolved.

Weigh entire amount of velagliflozin into a beaker and add in portions to stirred solution.

Stir until fully dissolved.

Use an 8 µm filter to filtrate the solution

The solutions were found to have the following characteristics (Table 5).

**Table 5:**

| Formulation / Solution | LogP-Parameter [-] (Eq. 4) | Turbidity [NTU] | Density* [g/mL] | Appearance |
|---|---|---|---|---|
| Composition 1 | -1.45 | 1.0 | 1.105 | yellowish, clear solution, no particles |
| Composition 2 | -1.43 | 1.4 | 1.108 | yellowish, clear solution, no particles |
| Composition 3 | -1.59 | 1.4 | 1.073 | yellowish, clear solution, no particles |
| Composition 4 | -1.33 | 0.8 | 1.108 | yellowish, clear solution, no particles |

| | | | | |
|---|---|---|---|---|
| *) Density was measured using an Anton Paar DMA38 density meter | | | | |

Furthermore, the efficacy of antimicrobial preservation of Compositions 1 to 4 was investigated. The testing criteria applied are those for evaluation of antimicrobial activity for oral preparations according to Pharm. Eur. 7 (tests at 14 days and 28 days). The acceptance criteria of the Ph. Eur. 7, Method 5.1.3 "Efficacy of Antimicrobial Preservation", and USP 34, Method <51> "Antimicrobial Effectiveness Testing" are listed in the following Table 6.

**Table 6: Criteria for evaluation of antimicrobial activity for oral preparations according to Pharm. Eur. 7 and USP 34**

| Type of microorganism | Ph. Eur. 7 Method 5.1.3. | | USP 34 Method <51> | |
|---|---|---|---|---|
| | Logarithmic reduction of microorganisms after | | | |
| | 14 days | 28 days | 14 days | 28 days |
| Bacteria | > 3 | No increase from 14 days ¹⁾ | > 1.0 | No increase from 14 days ²⁾ |
| Funghi | > 1 | No increase from 14 days¹⁾ | No increase from initial calc. count ²⁾ | No increase from initial calc. count ²⁾ |

| | | | | |
|---|---|---|---|---|
| ¹⁾ for Ph Eur: No increase = no increase in number ²⁾ for USP: No increase = not more than 0.5 log₁₀ units higher than reference value | | | | |

The following microorganisms were tested: Pseudomonas aeruginosa, Staphylococcus aureus, Escherichia coli, Candida albicans, Aspergillus brasiliensis, Burkholderia cepacia.

In the performed tests the USP 34 Method <51> Criteria as listed in Table 6 were found to be fulfilled for all solutions for all microorganisms.

### Example 5

After sample preparation the solution was filled in polyethylene bottles and closed with polypropylene caps for chemical stability investigations.

For the Reference Compositions 1, 2, 3 and 4 the API content stays constant as 15.0 ± 0.1 mg/mL and no additional degradation ≥1.0% was measured by HPLC analytics after 6 months at 25°C/60% r.h and 40°C/75% r.h. (table 7).

**Table 7**

| | Assay [mg/mL] / degradation [%] measured by HPLC | |
|---|---|---|
| Composition | 6 months at 25°C/60% r.h. | 6 months at 40°C/75% r.h. |
| 1 | 15.1 / <1.0% | 15.1 / <1.0% |
| 2 | 15.1 / <1.0% | 15.1 / <1.0% |
| 3 | 15.0 / <1.0% | 14.9 / <1.0% |
| 4 | 15.1 / <1.0% | 15.1 / <1.0% |

### Example 6

Formulation samples were produced with compositions listed in the following Tables 8 and 9.

**Table 8: Exemplary pharmaceutical compositions according to the present invention**

| Ingredient | Composition 5 [mg/mL] | Composition 6 [mg/mL] | Composition 7 [mg/mL] | Composition 8 [mg/mL] |
|---|---|---|---|---|
| Velagliflozin | 15.0 | 15.0 | 15.0 | 15.0 |
| Citric Acid Monohydrate | 9.8 | 9.8 | 9.8 | 9.8 |
| NaOH 1 M | 46.4 | 46.4 | 46.4 | 46.4 |
| Kollidon 12 | - | 100.0 | 50.0 | - |
| PEG 300 | 150.0 | - | 100.0 | - |
| Sodium dodecyl sulphate (SDS; Na-Lauryl-Sulfate) | - | - | - | 50.0 |
| Sodium benzoate | 2.0 | 2.0 | 2.0 | 2.0 |
| Na-metabisulfite | - | 2.0 | 2.0 | - |
| Sorbitol | 170.0 | 170.0 | 170.0 | 170.0 |
| Honey Flavor | 1.5 | 1.5 | 1.5 | 1.5 |
| Water, purified | ad. 1100 ml | ad. 1100 ml | ad. 1100 ml | Ad. 1100 ml |

**Table 9: Exemplary pharmaceutical compositions according to the present invention**

| Ingredient | Composition 9 [mg/mL] | Composition 10 [mg/mL] | Composition 11 [mg/mL] | Composition 12 [mg/mL] |
|---|---|---|---|---|
| Velagliflozin | 15.0 | 15.0 | 15.0 | 15.0 |
| Citric Acid Monohydrate | 9.8 | 9.8 | 9.8 | 9.8 |
| NaOH 1 M | 46.4 | 46.4 | 46.4 | 46.4 |
| Cremophor RH40 | 250.0 | - | - | - |
| Polysorbate 80 | - | 250.0 | - | - |
| Poloxamer 188 | - | - | 50.0 | - |
| PEG 400 | - | - | - | 200.0 |
| Sodium benzoate | 2.0 | 2.0 | 2.0 | 2.0 |
| Sorbitol | 170.0 | 170.0 | 170.0 | 170.0 |
| Honey Flavor | 1.5 | 1.5 | 1.5 | 1.5 |
| Water, purified | ad. 1100 ml | ad. 1100 ml | ad. 1100 ml | Ad. 1100 ml |

The following procedure was used to prepare the samples:
Weigh approximately 80% of expected amount of water into vessel.

Weigh entire amounts of NaOH 1N and citric acid monohydrate into a beaker and add to stirred water. Stir until fully dissolved.

Weigh entire amount of respective solubilizers (Kollidon 12, PEG 300, Na-Lauryl-Sulfate, Cremophor RH40, Polysorbate 80, Poloxamer 188 and/or PEG 400) into a beaker and add slowly to stirred solution.

Weigh antioxidant (if needed) and preservative and add to the solution under stirring.

Weigh entire amount of sweetener and/or viscosity enhancer and add to the solution under stirring.

Weigh entire amount of flavour into a beaker and add to stirred solution. Stir until fully dissolved.

Weigh entire amount of velagliflozin into a beaker and add in portions to stirred solution.

Stir until fully dissolved.

Fill up to 1100 ml with water.

Stir until a homogeneous, clear solution is reached.

A temperature test (alternating storage between 4°C and 23°C, over 3 weeks) showed no visual changes for all samples (Table 10).

**Table 10**

| Short-term storage test over 3 weeks with alternating storage at 4°C and room temperature (23°C) - visual inspection | |
|---|---|
| | **Appearance** |
| **Composition 5** | Visually no noticeable changes after storage; No precipitation, crystallization or color change recognizable. |
| **Composition 6** | Visually no noticeable changes after storage; No precipitation, crystallization or color change recognizable. |
| **Composition 7** | Visually no noticeable changes after storage; No precipitation, crystallization or color change recognizable. |
| **Composition 8** | Visually no noticeable changes after storage; No precipitation, crystallization or color change recognizable. |
| **Composition 9** | Visually no noticeable changes after storage; No precipitation, crystallization or color change recognizable. |
| **Composition 10** | Visually no noticeable changes after storage; No precipitation, crystallization or color change recognizable. |
| **Composition 11** | Visually no noticeable changes after storage; No precipitation, crystallization or color change recognizable. |
| **Composition 12** | Visually no noticeable changes after storage; No precipitation, crystallization or color change recognizable. |

## Claims

1. An aqueous pharmaceutical composition comprising at least one SGLT-2 inhibitor and one or more solubilizing agents, wherein:
(i) the at least one SGLT-2 inhibitor is velagliflozin,
(ii) velagliflozin is the only SGLT-2 inhibitor contained in such aqueous pharmaceutical composition,
(iii) such aqueous pharmaceutical composition is substantially free of organic solvents, preferably does not contain any organic solvents, and
(iv) the one or more solubilizing agents are selected from the group consisting of: surfactants, anionic surfactants, non-ionic surfactants, hydrogenated castor oils, polyoxyethylene-polyoxypropylene block copolymers, polyethylene glycols, propylene glycol derivatives, polyvinylpyrrolidones, preferably selected from the group consisting of: Sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate, Kollidon 12 (povidone).

2. The aqueous pharmaceutical composition according to claim 1, wherein the aqueous pharmaceutical composition is for, preferably direct, administration to a subject, preferably without further mandatory processing and/or purification steps, more preferably an animal, even more preferably a mammal, in particular a horse, cat, dog or cow and/or wherein the aqueous pharmaceutical composition is sterile.

3. The aqueous pharmaceutical composition according to any one of claims 1 to 2, wherein the total amount of the one or more solubilizing agents is from 1 to 50 g/100 mL (1 - 50 % w/v), more preferably from 1 to 45 g/100 mL (1 - 45 % w/v), even more preferably from 1 to 40 g/100 mL (1 - 40 % w/v), even more preferably from 1 to 35 g/100 mL (1 - 35 % w/v), even more preferably from 1 to 30 g/100 mL (1 - 30 % w/v), even more preferably from 1 to 25 g/100 mL (1 - 25 % w/v), most preferably from 5 to 25 g/100 mL (5 - 25 % w/v).

4. The aqueous pharmaceutical composition according to any one of claims 1 to 3, wherein two or more solubilizing agents are contained in the aqueous pharmaceutical composition.

5. The aqueous pharmaceutical composition according to claim 4, wherein two solubilizing agents are contained in such aqueous pharmaceutical composition, wherein preferably the amount of the first solubilizing agent and the amount of the second solubilizing agent are independently from each other selected from 1 to 50 g/100 mL (1 - 50 % w/v), more preferably from 1 to 45 g/100 mL (1 - 45 % w/v), even more preferably from 1 to 40 g/100 mL (1 - 40 % w/v), even more preferably from 1 to 35 g/100 mL (1 - 35 % w/v), even more preferably from 1 to 30 g/100 mL (1 - 30 % w/v), even more preferably from 1 to 25 g/100 mL (1 - 25 % w/v), most preferably from 5 to 25 g/100 mL (5 - 25 % w/v).

6. The aqueous pharmaceutical composition according to claim 5, wherein the two solubilizing agents are independently from each other selected from the group consisting of: sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate, Kollidon 12 (povidone), preferably wherein such two solubilizing agents are Kollidon 12 (povidone) as well as PEG 200, PEG 300 or PEG 400, more preferably wherein such two solubilizing agents are Kollidon 12 (povidone) and PEG 300.

7. The aqueous pharmaceutical composition according to any one of claims 1 to 6, wherein the aqueous pharmaceutical composition is a solution, an emulsion or a suspension, preferably a solution, an emulsion or a suspension with an NTU value of equal to or less than 10.0, more preferably equal to or less than 7.0, even more preferably equal to or less than 30, wherein preferably the aqueous pharmaceutical composition is a solution with an NTU value of equal to or less than 3.0.

8. The aqueous pharmaceutical composition according to any one of claims 1 to 7, wherein such aqueous pharmaceutical composition contains from 30 to 100 g/100 mL (30 to 100 % w/v), preferably from 30 to 95 g/100 mL (30 to 95 % w/v), more preferably from 30 to 90 g/100 mL (30 to 90 % w/v), more preferably from 30 to 85 g/100 mL (30 to 85 % w/v), even more preferably from 30 to 80 g/100 mL (30 to 80 % w/v), most preferably from 50 to 80 g/100 mL (50 to 80 % w/v) water, more preferably in the form of aqueous buffer, such as citric acid buffer.

9. The aqueous pharmaceutical composition according to claim 8, wherein such aqueous pharmaceutical composition has a pH value of from 2 to 7, preferably from 3 to 7, more preferably from 3.0 to 6.5, even more preferably from 4.0 to 6.5, even more preferably from 4.0 to 5.0, and most preferably of 4.5.

10. The aqueous pharmaceutical composition according to any one of claims 1 to 9, wherein such aqueous pharmaceutical composition additionally comprises one or more preservatives, preferably selected from the group consisting of: sorbic acid or salts thereof, more preferably sodium sorbate, potassium sorbate, calcium sorbate; benzoic acid or salts thereof, more preferably sodium benzoate; benzalkonium chloride; benzethonium chloride; benzyl alcohol, cetylpyridinium chloride; sodium metabisulfite; sodium acetate; parabens and salts thereof, more preferably methylparaben, ethylparaben, propylparaben, butylparaben, butylparaben sodium; most preferably benzoic acid and/or salts thereof, such as sodium benzoate.

11. The aqueous pharmaceutical composition according to any one of claims 1 to 10, wherein such aqueous pharmaceutical composition additionally comprises one or more antioxidation agents, preferably selected from the group consisting of: ascorbic acid or pharmaceutically acceptable salts thereof, particularly sodium ascorbate; citric acid (anhydrous and/or monohydrate) or pharmaceutically acceptable salts thereof, more preferably sodium citrate; erythorbic acid; fumaric acid; malic acid; monothioglycerol; phosphoric acid; sodium metabisulfite; potassium metabisulfite; propionic acid; sodium bisulfite; sodium sulfite; resveratrol; butylhydroxyanisol; butylhydroxytoluene; gallate derivatives, particularly propylgallate, octylgallate; Vitamin E or pharmaceutically acceptable salts thereof; ascorbyl palmitate; edetic acid or pharmaceutically acceptable salts thereof; most preferably sodium metabisulfite.

12. The aqueous pharmaceutical composition according to any one of claims 1 to 11, wherein such aqueous pharmaceutical composition additionally comprises one or more viscosity-enhancing agents, preferably selected from the group consisting of: inorganic gel forming agents, organic gel forming agents, cellulose derivatives, more preferably selected from the group consisting of: hydroxyl ethyl cellulose, hydroxyl propyl methyl cellulose, silicon dioxide.

13. The aqueous pharmaceutical composition according to any one of claims 1 to 12, wherein such aqueous pharmaceutical composition additionally comprises one or more flavours and/or sweeteners, preferably selected from the group consisting of: honey flavor, lime/salvia flavor, jasmine flavor, lavender flavor, peppermint flavor, raspberry flavor, lemon flavor, herbs flavor, meat flavor, vanillin/vanilla flavor, saccharine, aspartame, sorbitol, xylitol.

14. The aqueous pharmaceutical composition according to any one of claims 1 to 13 comprising, preferably consisting of:
(i) velagliflozin as single SGLT-2 inhibitor;
(ii) optionally, but preferred, one or more preservatives; preferably selected from the group consisting of: sorbic acid or salts thereof, preferably sodium sorbate, potassium sorbate, calcium sorbate; benzoic acid or salts thereof, preferably sodium benzoate; benzalkonium chloride; benzethonium chloride; benzyl alcohol, cetylpyridinium chloride; sodium metabisulfite; sodium acetate; parabens and salts thereof, preferably methylparaben, ethylparaben, propylparaben, butylparaben, butylparaben sodium; or combinations thereof; most preferably benzoic acid and/or salts thereof, such as sodium benzoate;
(iii) optionally, one or more flavors and/or sweeteners, preferably selected from the group consisting of: honey flavor, lime/salvia flavor, jasmine flavor, lavender flavor, peppermint flavor, raspberry flavor, lemon flavor, herbs flavor, meat flavor, vanillin/vanilla flavor, saccharine, aspartame, sorbitol, xylitol;
(iv) water, preferably in the form of aqueous buffer, more preferably citric acid buffer, even more preferably aqueous buffer with pH 4.5, most preferably citric acid buffer with pH 45;
(v) one or more solubilizing agents, preferably selected from the group consisting of: surfactants, anionic surfactants, non-ionic surfactants, hydrogenated castor oils, polyoxyethylene-polyoxypropylene block copolymers, polyethylene glycols, propylene glycol derivatives, polyvinylpyrrolidones, more preferably selected from the group consisting of: sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate, Kollidon 12 (povidone);
(vi) optionally, one or more viscosity-enhancing agents, preferably selected from the group consisting of: inorganic gel forming agents, organic gel forming agents, cellulose derivatives, more preferably selected from the group consisting of: hydroxyl ethyl cellulose, hydroxyl propyl methyl cellulose, silicon dioxide;
(vii) optionally, one or more antioxidation agents, preferably selected from the group consisting of: ascorbic acid or pharmaceutically acceptable salts thereof, particularly sodium ascorbate; citric acid (anhydrous and/or monohydrate) or pharmaceutically acceptable salts thereof, more preferably sodium citrate; erythorbic acid; fumaric acid; malic acid; monothioglycerol; phosphoric acid; sodium metabisulfite; potassium metabisulfite; propionic acid; sodium bisulfite; sodium sulfite; resveratrol; butylhydroxyanisol; butylhydroxytoluene; gallate derivatives, particularly propylgallate, octylgallate; Vitamin E or pharmaceutically acceptable salts thereof; ascorbyl palmitate; edetic acid or pharmaceutically acceptable salts thereof; most preferably sodium metabisulfite.

15. The aqueous pharmaceutical composition according to any one of claims 1 to 14 comprising, preferably consisting of:
(i) 0.5 - 20.0 g/100 mL (0.5 - 20.0 % w/v), preferably 0.5 - 15.0 g/100 mL (0.5 - 15.0 % w/v), more preferably 1.0 -1.5 g/100 mL (1.0 - 1.5 % w/v) velagliflozin as single SGLT-2 inhibitor;
(ii) 0 - 3.0 g/100 mL (0 - 3.0 % w/v), preferably 0.05 - 3.0 g/100 mL (0.05 - 3.0 % w/v), more preferably 0.05 - 2.0 g/100 mL (0.05 - 2.0 % w/v) one or more preservatives; more preferably selected from the group consisting of: sorbic acid or salts thereof, preferably sodium sorbate, potassium sorbate, calcium sorbate; benzoic acid or salts thereof, preferably sodium benzoate; benzalkonium chloride; benzethonium chloride; benzyl alcohol, cetylpyridinium chloride; sodium metabisulfite; sodium acetate; parabens and salts thereof, preferably methylparaben, ethylparaben, propylparaben, butylparaben, butylparaben sodium; or combinations thereof; most preferably benzoic acid and/or salts thereof, such as sodium benzoate;
(iii) 0 - 40 g/100 mL (0 - 40 % w/v), preferably 0 - 30 g/100 mL (0 - 30 % w/v), more preferably 0 - 2 g/100 mL (0 - 2 % w/v) one or more flavors and/or sweeteners, more preferably selected from the group consisting of: honey flavor, lime/salvia flavor, jasmine flavor, lavender flavor, peppermint flavor, raspberry flavor, lemon flavor, herbs flavor, meat flavor, vanillin/vanilla flavor, saccharine, aspartame, sorbitol, xylitol;
(iv) 30 to 100 g/100 mL (30 to 100 % w/v), preferably from 30 to 95 g/100 mL (30 to 95 % w/v), more preferably from 30 to 90 g/100 mL (30 to 90 % w/v), more preferably from 30 to 85 g/100 mL (30 to 85 % w/v), even more preferably from 30 to 80 g/100 mL (30 to 80 % w/v), most preferably from 50 to 80 g/100 mL (50 to 80 % w/v) water, preferably in the form of aqueous buffer, more preferably citric acid buffer, even more preferably aqueous buffer with pH 4.5, most preferably citric acid buffer with pH 4.5;
(v) 1 to 50 g/100 mL (1 - 50 % w/v), preferably from 1 to 45 g/100 mL (1 - 45 % w/v), even more preferably from 1 to 40 g/100 mL (1 - 40 % w/v), even more preferably from 1 to 35 g/100 mL (1 - 35 % w/v), even more preferably from 1 to 30 g/100 mL (1 - 30 % w/v), even more preferably from 1 to 25 g/100 mL (1 - 25 % w/v), most preferably from 5 to 25 g/100 mL (5 - 25 % w/v) one or more solubilizing agents, preferably selected from the group consisting of: surfactants, anionic surfactants, non-ionic surfactants, hydrogenated castor oils, polyoxyethylene-polyoxypropylene block copolymers, polyethylene glycols, propylene glycol derivatives, polyvinylpyrrolidones, more preferably selected from the group consisting of: sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate, Kollidon 12 (povidone);
(vi) 0 - 40 g/100 mL (0 - 40 % w/v), preferably 10 - 30 g/100 mL (10 - 30 % w/v) one or more viscosity-enhancing agents, preferably selected from the group consisting of: inorganic gel forming agents, organic gel forming agents, cellulose derivatives, more preferably selected from the group consisting of: hydroxyl ethyl cellulose, hydroxyl propyl methyl cellulose, silicon dioxide;
(vii) 0 - 1.0 g/100 mL (0 - 1.0 % w/v), preferably 0 - 0.5 g/100 mL (0 - 0.5 % w/v), more preferably 0.1 - 0.3 g/100 mL (0.1 - 0.3 % w/v) one or more antioxidation agents, preferably selected from the group consisting of: ascorbic acid or pharmaceutically acceptable salts thereof, particularly sodium ascorbate; citric acid (anhydrous and/or monohydrate) or pharmaceutically acceptable salts thereof, more preferably sodium citrate; erythorbic acid; fumaric acid; malic acid; monothioglycerol; phosphoric acid; sodium metabisulfite; potassium metabisulfite; propionic acid; sodium bisulfite; sodium sulfite; resveratrol; butylhydroxyanisol; butylhydroxytoluene; gallate derivatives, particularly propylgallate, octylgallate; Vitamin E or pharmaceutically acceptable salts thereof; ascorbyl palmitate; edetic acid or pharmaceutically acceptable salts thereof; most preferably sodium metabisulfite.

16. The aqueous pharmaceutical composition according to any one of claims 1 to 15 comprising, preferably consisting of:
velagliflozin; sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate and/or Kollidon 12 (povidone); sodium benzoate; optionally, Na-metabisulfite; water;
or
velagliflozin; citric acid monohydrate; NaOH; sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate and/or Kollidon 12 (povidone); sodium benzoate; optionally, Na-metabisulfite; water;
or
velagliflozin; citric acid monohydrate; NaOH; sodium dodecyl sulphate (SDS), Cremophor RH 40 (PEG-40 Hydrogenated Castor Oil, Macrogol glycerol hydroxystearate 40), polysorbate 20, Lutrol F 68 (Poloxamer 188), PEG 200, PEG 300, PEG 400, propylene glycol monolaurate and/or Kollidon 12 (povidone); sodium benzoate; optionally, Na-metabisulfite; sorbitol and/or xylitol; honey flavor and/or vanillin and/or meat flavor; water.

17. The aqueous pharmaceutical composition according to any one of claims 1 to 16 selected from the group consisting of the following compositions 5 to 12:
| Ingredient | Composition 5 [mg/mL] | Composition 6 [mg/mL] | Composition 7 [mg/mL] | Composition 8 [mg/mL] |
|---|---|---|---|---|
| Velagliflozin | 15.0 | 15.0 | 15.0 | 15.0 |
| Citric Acid Monohydrate | 9.8 | 9.8 | 9.8 | 9.8 |
| NaOH 1 M | 46.4 | 46.4 | 46.4 | 46.4 |
| Kollidon 12 | - | 100.0 | 50.0 | - |
| PEG 300 | 150.0 | - | 100.0 | - |
| Sodium dodecyl sulphate (SDS; Na-Lauryl-Sulfate) | - | - | - | 50.0 |
| Sodium benzoate | 2.0 | 2.0 | 2.0 | 2.0 |
| Na-metabisulfite | - | 2.0 | 2.0 | - |
| Sorbitol | 170.0 | 170.0 | 170.0 | 170.0 |
| Honey Flavor | 1.5 | 1.5 | 1.5 | 1.5 |
| Water, purified | ad. 1100 ml | ad. 1100 ml | ad. 1100 ml | ad. 1100 ml |
| Ingredient | Composition 9 [mg/mL] | Composition 10 [mg/mL] | Composition 11 [mg/mL] | Composition 12 [mg/mL] |
|---|---|---|---|---|
| Velagliflozin | 15.0 | 15.0 | 15.0 | 15.0 |
| Citric Acid Monohydrate | 9.8 | 9.8 | 9.8 | 9.8 |
| NaOH 1 M | 46.4 | 46.4 | 46.4 | 46.4 |
| Cremophor RH40 | 250.0 | - | - | - |
| Polysorbate 80 | - | 250.0 | - | - |
| Poloxamer 188 | - | - | 50.0 | - |
| PEG 400 | - | - | - | 200.0 |
| Sodium benzoate | 2.0 | 2.0 | 2.0 | 2.0 |
| Sorbitol | 170.0 | 170.0 | 170.0 | 170.0 |
| Honey Flavor | 1.5 | 1.5 | 1.5 | 1.5 |
| Water, purified | ad. 1100 ml | ad. 1100 ml | ad. 1100 ml | ad. 1100 ml |

18. The aqueous pharmaceutical composition according to any one of claims 1 to 17, wherein such aqueous pharmaceutical composition is for oral and/or parenteral administration, preferably oral administration.

19. The aqueous pharmaceutical composition according to any one of claims 1 to 18 for use in a method of treating and/or preventing one or more medicinal indications in a subject in need of such treatment and/or prevention, preferably an animal, more preferably a mammal, in particular a horse, cat, dog or cow, selected from among the medicinal indications:
(i) a metabolic disorder of an equine animal, wherein preferably the metabolic disorder is one or more disorders selected from insulin resistance, hyperinsulinemia, impaired glucose tolerance, dyslipidemia, dysadipokinemia, subclinical inflammation, systemic inflammation, low grade systemic inflammation, obesity, and/or regional adiposity, wherein preferably the metabolic disorder is insulin resistance, hyperinsulinemia, and/or a clinical condition associated with insulin resistance and/or hyperinsulinemia, wherein preferably said clinical condition is one or more conditions selected from impaired glucose tolerance, dyslipidemia, dysadipokinemia, subclinical inflammation, systemic inflammation, low grade systemic inflammation, obesity, and/or regional adiposity;
(ii) a metabolic disorder of an equine animal, wherein the metabolic disorder is one or more disorders selected from laminitis, vascular dysfunction, hypertension, hepatic lipidosis, atherosclerosis, hyperadrenocorticism, Pituitary Pars Intermedia Dysfunction and/or Equine Metabolic Syndrome, wherein preferably the metabolic disorder is a clinical condition/sign associated with insulin resistance and/or hyperinsulinemia, wherein said clinical condition/sign preferably is one or more conditions selected from laminitis, vascular dysfunction, hypertension, hepatic lipidosis, atherosclerosis, hyperadrenocorticism, Pituitary Pars Intermedia Dysfunction and/or Equine Metabolic Syndrome;
(iii) a metabolic disorder of a feline animal, wherein preferably the metabolic disorder is one or more selected from the group consisting of: ketoacidosis, pre-diabetes, diabetes mellitus type 1 or type 2, insulin resistance, acromegaly, diabetes with elevated IGF-1 concentration, obesity, hyperglycemia, impaired glucose tolerance, hyperinsulinemia, dyslipidemia, dysadipokinemia, subclinical inflammation, systemic inflammation, low grade systemic inflammation, hepatic lipidosis, atherosclerosis, inflammation of the pancreas, neuropathy and/or Syndrome X (metabolic syndrome) and/or loss of pancreatic beta cell function and/or wherein the remission of the metabolic disorder, preferably diabetic remission, is achieved and/or maintained;
(iv) a metabolic disorder of a canine animal, wherein preferably the metabolic disorder is one or more selected from the group consisting of: ketoacidosis, pre-diabetes, insulin dependent diabetes mellitus, insulin resistance diabetes, insulin resistance, obesity, hyperglycemia, hyperglycemia induced cataract formation, impaired glucose tolerance, hyperinsulinemia, dyslipidemia, dysadipokinemia, subclinical inflammation, systemic inflammation, low grade systemic inflammation, hepatic lipidosis, inflammation of the pancreas, metabolic disorder consequences, such as hypertension, renal dysfunction and/or musculoskeletal disorders, and/or Syndrome X (metabolic syndrome), preferably pre-diabetes, insulin dependent diabetes mellitus, insulin resistance diabetes, insulin resistance, wherein preferably the development of hyperglycemia induced cataract formation is prevented or remission is achieved and/or wherein preferably the development of metabolic disorder consequences, such as hypertension, renal dysfunction and/or musculoskeletal disorders, is prevented or progression is slowed or remission is achieved.
(v) a cardiac disease of a feline animal, wherein preferably the cardiac disease is one or more selected from the group consisting of: heart failure, heart failure due to one or more cardiomyopathies, heart failure due to hypertrophic cardiomyopathy (HCM), heart failure due to restrictive cardiomyopathy (RCM), heart failure due to dilated cardiomyopathy (DCM), heart failure due to unclassified cardiomyopathy (UCM), heart failure due to arrhythmogenic right ventricular cardiomyopathy (ARVC), hypertrophic cardiomyopathy (HCM), restrictive cardiomyopathy (RCM), dilated cardiomyopathy (DCM), unclassified cardiomyopathy (UCM), and/or arrhythmogenic right ventricular cardiomyopathy (ARVC); preferably selected from the group consisting of: heart failure due to one or more cardiomyopathies, heart failure due to hypertrophic cardiomyopathy (HCM), hypertrophic cardiomyopathy (HCM),
(vi) drying-off of a non-human mammal, preferably ruminant, improving and/or facilitating the drying-off of a non-human mammal, preferably ruminant, reducing the milk production, preferably milk production and/or secretion, in a pregnant and/or lactating non-human mammal, preferably ruminant, decreasing milk accumulation and/or engorgement in the udder, preferably udder and/or mammary gland, of a non-human mammal, preferably ruminant, decreasing the discomfort associated with udder engorgement, such as increasing the daily lying time and/or reduction of stress, of a non-human mammal, preferably ruminant, decreasing milk leakage after drying-off of a non-human mammal, preferably ruminant, decreasing the incidence of intra-mammary infections (IMI), preferably mastitis and/or metritis, in a non-human mammal, preferably ruminant;
(vii) a cardiac disease of a non-human mammal, excluding a feline, in particular a canine, wherein preferably the cardiac disease is one or more selected from the group consisting of: heart failure; congestive heart failure; asymptomatic / preclinical / occult heart failure; heart failure due to (myxomatous) mitral valve disease [(M)MVD]; congestive heart failure due to (myxomatous) mitral valve disease [(M)MVD]; asymptomatic /preclinical / occult heart failure due to (myxomatous) mitral valve disease [(M)MVD]; (myxomatous) mitral valve disease [(M)MVD]; clinically overt (myxomatous) mitral valve disease [(M)MVD]; asymptomatic / preclinical / occult (myxomatous) mitral valve disease [(M)MVD]; heart failure due to dilated cardiomyopathy (DCM); congestive heart failure due to dilated cardiomyopathy (DCM); asymptomatic / preclinical / occult heart failure due to dilated cardiomyopathy (DCM); dilated cardiomyopathy (DCM); clinically overt dilated cardiomyopathy (DCM); asymptomatic / preclinical / occult dilated cardiomyopathy (DCM); aortic stenosis (valvular, supravalvular and/or subvalvular);
(viii) hypertension in a non-human mammal, preferably a carnivore, more preferably a cat or a dog, wherein preferably the hypertension is one or more selected from the group consisting of: situational hypertension, secondary hypertension and idiopathic hypertension, wherein preferably the secondary hypertension is selected from the group consisting of hypertension associated with chronic kidney disease (CKD), diabetes, obesity, heart disease, endocrine disease, such as Cushing's disease, hyperthyroidism, acromegaly, and elevated blood pressure (BP) induced by medicaments, preferably by glucocorticoids, mineralocorticoids, erythropoiesis-stimulating agents, ephedrine and/or high dose sodium chloride;
(ix) a renal disease of a non-human mammal, preferably a carnivore, more preferably a cat or a dog, wherein preferably the renal disease is one or more selected from the group consisting of: renal dysplasia, glomerulopathy, polycystic kidney disease, amyloidosis, tubulo-nephritis / tubulointerstitial nephritis (TIN), acute kidney disease, chronic kidney disease.

20. A process for producing the aqueous pharmaceutical composition according to any one of claims 1 to 18, comprising the steps (in any meaningful order):
(i) water is used as starting material;
(ii) adding one or more solubilizing agents to water resulting from step (i);
(iii) dissolving velagliflozin in the mixture resulting from step (ii);
(iv) optionally, dissolving the one or more preservatives in the mixture resulting from step (iii),
(v) optionally, further dissolving further excipients, such as pH modifier(s), flavor(s), sweeteners, antioxidation agents, viscosity-enhancing agents and the like, in the mixture resulting from step (iii) or optionally (iv);
(vi) optionally, filtrating the mixture resulting from step (iii), optionally step (iv) or optionally step (v); whereby, optionally, independently from each other after any of the individual process steps - be they mandatory or optional - an additional mixing step is performed.

21. A kit-of-parts comprising:
(a) an aqueous pharmaceutical composition according to any one of claims 1 to 18; and
(b) a package leaflet including the information that the aqueous pharmaceutical composition is to be used for the prevention and/or treatment of one or more medicinal indications in a subject in need of such prevention and/or treatment, which are selected from among the medicinal indications:
(i) a metabolic disorder of an equine animal, wherein preferably the metabolic disorder is one or more disorders selected from insulin resistance, hyperinsulinemia, impaired glucose tolerance, dyslipidemia, dysadipokinemia, subclinical inflammation, systemic inflammation, low grade systemic inflammation, obesity, and/or regional adiposity, wherein preferably the metabolic disorder is insulin resistance, hyperinsulinemia, and/or a clinical condition associated with insulin resistance and/or hyperinsulinaemia; wherein preferably said clinical condition is one or more conditions selected from impaired glucose tolerance, dyslipidemia, dysadipokinemia, subclinical inflammation, systemic inflammation, low grade systemic inflammation, obesity, and/or regional adiposity;
(ii) a metabolic disorder of an equine animal, wherein the metabolic disorder is one or more disorders selected from laminitis, vascular dysfunction, hypertension, hepatic lipidosis, atherosclerosis, hyperadrenocorticism, Pituitary Pars Intermedia Dysfunction and/or Equine Metabolic Syndrome, wherein preferably the metabolic disorder is a clinical condition/sign associated with insulin resistance and/or hyperinsulinaemia, wherein said clinical condition/sign preferably is one or more conditions selected from laminitis, vascular dysfunction, hypertension, hepatic lipidosis, atherosclerosis, hyperadrenocorticism, Pituitary Pars Intermedia Dysfunction and/or Equine Metabolic Syndrome;
(iii) a metabolic disorder of a feline animal, wherein preferably the metabolic disorder is one or more selected from the group consisting of: ketoacidosis, pre-diabetes, diabetes mellitus type 1 or type 2, insulin resistance, acromegaly, diabetes with elevated IGF-1 concentration, obesity, hyperglycemia, impaired glucose tolerance, hyperinsulinemia, dyslipidemia, dysadipokinemia, subclinical inflammation, systemic inflammation, low grade systemic inflammation, hepatic lipidosis, atherosclerosis, inflammation of the pancreas, neuropathy and/or Syndrome X (metabolic syndrome) and/or loss of pancreatic beta cell function and/or wherein the remission of the metabolic disorder, preferably diabetic remission, is achieved and/or maintained;
(iv) a metabolic disorder of a canine animal, wherein preferably the metabolic disorder is one or more selected from the group consisting of: ketoacidosis, pre-diabetes, insulin dependent diabetes mellitus, insulin resistance diabetes, insulin resistance, obesity, hyperglycemia, hyperglycemia induced cataract formation, impaired glucose tolerance, hyperinsulinemia, dyslipidemia, dysadipokinemia, subclinical inflammation, systemic inflammation, low grade systemic inflammation, hepatic lipidosis, inflammation of the pancreas, metabolic disorder consequences, such as hypertension, renal dysfunction and/or musculoskeletal disorders, and/or Syndrome X (metabolic syndrome), preferably pre-diabetes, insulin dependent diabetes mellitus, insulin resistance diabetes, insulin resistance, wherein preferably the development of hyperglycemia induced cataract formation is prevented or remission is achieved and/or wherein preferably the development of metabolic disorder consequences, such as hypertension, renal dysfunction and/or musculoskeletal disorders, is prevented or progression is slowed or remission is achieved.
(v) a cardiac disease of a feline animal, wherein preferably the cardiac disease is one or more selected from the group consisting of: heart failure, heart failure due to one or more cardiomyopathies, heart failure due to hypertrophic cardiomyopathy (HCM), heart failure due to restrictive cardiomyopathy (RCM), heart failure due to dilated cardiomyopathy (DCM), heart failure due to unclassified cardiomyopathy (UCM), heart failure due to arrhythmogenic right ventricular cardiomyopathy (ARVC), hypertrophic cardiomyopathy (HCM), restrictive cardiomyopathy (RCM), dilated cardiomyopathy (DCM), unclassified cardiomyopathy (UCM), and/or arrhythmogenic right ventricular cardiomyopathy (ARVC); preferably selected from the group consisting of: heart failure due to one or more cardiomyopathies, heart failure due to hypertrophic cardiomyopathy (HCM), hypertrophic cardiomyopathy (HCM),
(vi) drying-off of a non-human mammal, preferably ruminant, improving and/or facilitating the drying-off of a non-human mammal, preferably ruminant, reducing the milk production, preferably milk production and/or secretion, in a pregnant and/or lactating non-human mammal, preferably ruminant, decreasing milk accumulation and/or engorgement in the udder, preferably udder and/or mammary gland, of a non-human mammal, preferably ruminant, decreasing the discomfort associated with udder engorgement, such as increasing the daily lying time and/or reduction of stress, of a non-human mammal, preferably ruminant, decreasing milk leakage after drying-off of a non-human mammal, preferably ruminant, decreasing the incidence of intra-mammary infections (IMI), preferably mastitis and/or metritis, in a non-human mammal, preferably ruminant;
(vii) a cardiac disease of a non-human mammal, excluding a feline, in particular a canine, wherein preferably the cardiac disease is one or more selected from the group consisting of: heart failure; congestive heart failure; asymptomatic / preclinical / occult heart failure; heart failure due to (myxomatous) mitral valve disease [(M)MVD]; congestive heart failure due to (myxomatous) mitral valve disease [(M)MVD]; asymptomatic /preclinical / occult heart failure due to (myxomatous) mitral valve disease [(M)MVD]; (myxomatous) mitral valve disease [(M)MVD]; clinically overt (myxomatous) mitral valve disease [(M)MVD]; asymptomatic / preclinical / occult (myxomatous) mitral valve disease [(M)MVD]; heart failure due to dilated cardiomyopathy (DCM); congestive heart failure due to dilated cardiomyopathy (DCM); asymptomatic / preclinical / occult heart failure due to dilated cardiomyopathy (DCM); dilated cardiomyopathy (DCM); clinically overt dilated cardiomyopathy (DCM); asymptomatic / preclinical / occult dilated cardiomyopathy (DCM); aortic stenosis (valvular, supravalvular and/or subvalvular);
(viii) hypertension in a non-human mammal, preferably a carnivore, more preferably a cat or a dog, wherein preferably the hypertension is one or more selected from the group consisting of: situational hypertension, secondary hypertension and idiopathic hypertension, wherein preferably the secondary hypertension is selected from the group consisting of hypertension associated with chronic kidney disease (CKD), diabetes, obesity, heart disease, endocrine disease, such as Cushing's disease, hyperthyroidism, acromegaly, and elevated blood pressure (BP) induced by medicaments, preferably by glucocorticoids, mineralocorticoids, erythropoiesis-stimulating agents, ephedrine and/or high dose sodium chloride;
(ix) a renal disease of a non-human mammal, preferably a carnivore, more preferably a cat or a dog, wherein preferably the renal disease is one or more selected from the group consisting of: renal dysplasia, glomerulopathy, polycystic kidney disease, amyloidosis, tubulo-nephritis / tubulointerstitial nephritis (TIN), acute kidney disease, chronic kidney disease.

## Patentansprüche

1. Wässrige pharmazeutische Zusammensetzung, umfassend mindestens einen SGLT-2-Inhibitor und ein oder mehrere Lösungsvermittler, wobei:
(i) der mindestens eine SGLT-2-Inhibitor Velagliflozin ist,
(ii) Velagliflozin als einziger SGLT-2-Inhibitor in der wässrigen pharmazeutischen Zusammensetzung enthalten ist,
(iii) die wässrige pharmazeutische Zusammensetzung im Wesentlichen frei von organischen Lösungsmitteln ist, vorzugsweise keine organischen Lösungsmittel enthält, und
(iv) die ein oder mehreren Lösungsvermittler ausgewählt sind aus der Gruppe bestehend aus: Tensiden, anionischen Tensiden, nichtionischen Tensiden, hydrierten Ricinusölen, Polyoxyethylen-Polyoxypropylen-Blockcopolymeren, Polyethylenglykolen, Propylenglykolderivaten, Polyvinylpyrrolidonen, vorzugsweise ausgewählt sind aus der Gruppe bestehend aus: Natriumdodecylsulfat (SDS), Cremophor RH 40 (PEG-40 Hydrogenated Castor Oil, Macrogolglycerinhydroxystearat 40), Polysorbat 20, Lutrol F 68 (Poloxamer 188), PEG 200, PEG 300, PEG 400, Propylenglykolmonolaurat, Kollidon 12 (Povidon).

2. Wässrige pharmazeutische Zusammensetzung nach Anspruch 1, wobei die wässrige pharmazeutische Zusammensetzung zur, vorzugsweise direkten, Verabreichung an ein Individuum, bevorzugt ohne weitere obligatorische Verarbeitungs- und/oder Reinigungsschritte, bevorzugter an ein Tier, noch bevorzugter einen Säuger, insbesondere ein Pferd, eine Katze, einen Hund oder eine Kuh, bestimmt ist und/oder wobei die wässrige pharmazeutische Zusammensetzung steril ist.

3. Wässrige pharmazeutische Zusammensetzung nach einem der Ansprüche 1 bis 2, wobei die Gesamtmenge der ein oder mehreren Lösungsvermittler 1 bis 50 g/100 ml (1 - 50 % Gew./Vol.), bevorzugter 1 bis 45 g/100 ml (1 - 45 % Gew./Vol.), noch bevorzugter 1 bis 40 g/100 ml (1 - 40 % Gew./Vol.), noch bevorzugter 1 bis 35 g/100 ml (1 - 35 % Gew./Vol.), noch bevorzugter 1 bis 30 g/100 ml (1 - 30 % Gew./Vol.), noch bevorzugter 1 bis 25 g/100 ml (1 - 25 % Gew./Vol.), am meisten bevorzugt 5 bis 25 g/100 ml (5 - 25 % Gew./Vol.) beträgt.

4. Wässrige pharmazeutische Zusammensetzung nach einem der Ansprüche 1 bis 3, wobei zwei oder mehr Lösungsvermittler in der wässrigen pharmazeutischen Zusammensetzung enthalten sind.

5. Wässrige pharmazeutische Zusammensetzung nach Anspruch 4, wobei in dieser wässrigen pharmazeutischen Zusammensetzung zwei Lösungsvermittler enthalten sind, wobei vorzugsweise die Menge des ersten Lösungsvermittlers und die Menge des zweiten Lösungsvermittlers unabhängig voneinander ausgewählt sind aus 1 bis 50 g/100 ml (1 - 50 % Gew./Vol.), bevorzugter 1 bis 45 g/100 ml (1 - 45 % Gew./Vol.), noch bevorzugter 1 bis 40 g/100 ml (1 - 40 % Gew./Vol.), noch bevorzugter 1 bis 35 g/100 ml (1 - 35 % Gew./Vol.), noch bevorzugter 1 bis 30 g/100 ml (1 - 30 % Gew./Vol.), noch bevorzugter 1 bis 25 g/100 ml (1 - 25 % Gew./Vol.), am meisten bevorzugt 5 bis 25 g/100 ml (5 - 25 % Gew./Vol.).

6. Wässrige pharmazeutische Zusammensetzung nach Anspruch 5, wobei die zwei Lösungsvermittler unabhängig voneinander ausgewählt sind aus der Gruppe bestehend aus: Natriumdodecylsulfat (SDS), Cremophor RH 40 (PEG-40 Hydrogenated Castor Oil, Macrogolglycerinhydroxystearat 40), Polysorbat 20, Lutrol F 68 (Poloxamer 188), PEG 200, PEG 300, PEG 400, Propylenglykolmonolaurat, Kollidon 12 (Povidon), vorzugsweise wobei es sich bei den zwei Lösungsvermittlern um Kollidon 12 (Povidon) sowie PEG 200, PEG 300 oder PEG 400 handelt, bevorzugter wobei es sich bei den zwei Lösungsvermittlern um Kollidon 12 (Povidon) und PEG 300 handelt.

7. Wässrige pharmazeutische Zusammensetzung nach einem der Ansprüche 1 bis 6, wobei es sich bei der wässrigen pharmazeutische Zusammensetzung um eine Lösung, eine Emulsion oder eine Suspension, bevorzugt eine Lösung, eine Emulsion oder eine Suspension mit einem NTU-Wert gleich oder kleiner 10,0, bevorzugter gleich oder kleiner 7,0, noch bevorzugter gleich oder kleiner 3,0 handelt, wobei die wässrige pharmazeutische Zusammensetzung vorzugsweise eine Lösung mit einem NTU-Wert gleich oder kleiner 3,0 ist.

8. Wässrige pharmazeutische Zusammensetzung nach einem der Ansprüche 1 bis 7, wobei die wässrige pharmazeutische Zusammensetzung 30 bis 100 g/100 ml (30 bis 100 % Gew./Vol.), bevorzugt 30 bis 95 g/100 ml (30 bis 95 % Gew./Vol.), bevorzugter 30 bis 90 g/100 ml (30 bis 90 % Gew./Vol.), bevorzugter 30 bis 85 g/100 ml (30 bis 85 % Gew./Vol.), noch bevorzugter 30 bis 80 g/100 ml (30 bis 80 % Gew./Vol.), am meisten bevorzugt 50 bis 80 g/100 ml (50 bis 80 % Gew./Vol.) Wasser, bevorzugter in Form von wässrigem Puffer wie Citronensäurepuffer, enthält.

9. Wässrige pharmazeutische Zusammensetzung nach Anspruch 8, wobei die wässrige pharmazeutische Zusammensetzung einen pH-Wert von 2 bis 7, bevorzugt 3 bis 7, bevorzugter 3,0 bis 6,5, noch bevorzugter 4,0 bis 6,5, noch bevorzugter 4,0 bis 5,0 und am meisten bevorzugt von 4,5 aufweist.

10. Wässrige pharmazeutische Zusammensetzung nach einem der Ansprüche 1 bis 9, wobei die wässrige pharmazeutische Zusammensetzung zusätzlich ein oder mehrere Konservierungsmittel umfasst, bevorzugt ausgewählt aus der Gruppe bestehend aus: Sorbinsäure oder Salzen davon, bevorzugter Natriumsorbat, Kaliumsorbat, Calciumsorbat; Benzoesäure oder Salzen davon, bevorzugter Natriumbenzoat; Benzalkoniumchlorid; Benzethoniumchlorid; Benzylalkohol, Cetylpyridiniumchlorid; Natriumdisulfit; Natriumacetat; Parabenen und Salzen davon, bevorzugter Methylparaben, Ethylparaben, Propylparaben, Butylparaben, Butylparaben-Natrium; am meisten bevorzugt Benzoesäure und/oder Salzen davon wie Natriumbenzoat.

11. Wässrige pharmazeutische Zusammensetzung nach einem der Ansprüche 1 bis 10, wobei die wässrige pharmazeutische Zusammensetzung zusätzlich ein oder mehrere Antioxidationsmittel umfasst, bevorzugt ausgewählt aus der Gruppe bestehend aus: Ascorbinsäure oder pharmazeutisch unbedenklichen Salzen davon, besonders Natriumascorbat; Citronensäure (wasserfrei und/oder Monohydrat) oder pharmazeutisch unbedenklichen Salzen davon, bevorzugter Natriumcitrat; Erythorbinsäure; Fumarsäure; Äpfelsäure; Monothioglycerin; Phosphorsäure; Natriumdisulfit; Kaliumdisulfit; Propionsäure; Natriumhydrogensulfit; Natriumsulfit; Resveratrol, Butylhydroxyanisol; Butylhydroxytoluol; Gallatderivaten, besonders Propylgallat, Octylgallat; Vitamin E oder pharmazeutisch unbedenklichen Salzen davon; Ascorbylpalmitat; Edetinsäure oder pharmazeutisch unbedenklichen Salzen davon; am meisten bevorzugt Natriumdisulfit.

12. Wässrige pharmazeutische Zusammensetzung nach einem der Ansprüche 1 bis 11, wobei die wässrige pharmazeutische Zusammensetzung zusätzlich ein oder mehrere viskositätssteigernde Mittel umfasst, bevorzugt ausgewählt aus der Gruppe bestehend aus: anorganischen Gelbildnern, organischen Gelbildnern, Cellulosederivaten, bevorzugter ausgewählt aus der Gruppe bestehend aus: Hydroxylethylcellulose, Hydroxylpropylmethylcellulose, Siliciumdioxid.

13. Wässrige pharmazeutische Zusammensetzung nach einem der Ansprüche 1 bis 12, wobei die wässrige pharmazeutische Zusammensetzung zusätzlich ein oder mehrere Aromen und/oder Süßungsmittel umfasst, vorzugsweise ausgewählt aus der Gruppe bestehend aus: Honigaroma, Limetten-/Salbeiaroma, Jasminaroma, Lavendelaroma, Pfefferminzaroma, Himbeeraroma, Zitronenaroma, Kräuteraroma, Fleischgeschmack, Vanillin/Vanillearoma, Saccharin, Aspartam, Sorbit, Xylit.

14. Wässrige pharmazeutische Zusammensetzung nach einem der Ansprüche 1 bis 13, umfassend, vorzugsweise bestehend aus:
(i) Velagliflozin als einzigem SGLT-2-Inhibitor;
(ii) gegebenenfalls, aber bevorzugt, ein oder mehreren Konservierungsmitteln; vorzugsweise ausgewählt aus der Gruppe bestehend aus: Sorbinsäure oder Salzen davon, bevorzugt Natriumsorbat, Kaliumsorbat, Calciumsorbat; Benzoesäure oder Salzen davon, bevorzugt Natriumbenzoat; Benzalkoniumchlorid; Benzethoniumchlorid; Benzylalkohol, Cetylpyridiniumchlorid; Natriumdisulfit; Natriumacetat; Parabenen und Salzen davon, bevorzugt Methylparaben, Ethylparaben, Propylparaben, Butylparaben, Butylparaben-Natrium; oder Kombinationen davon; am meisten bevorzugt Benzoesäure und/oder Salzen davon wie Natriumbenzoat;
(iii) gegebenenfalls ein oder mehreren Aromen und/oder Süßungsmitteln, vorzugsweise ausgewählt aus der Gruppe bestehend aus: Honigaroma, Limetten-/Salbeiaroma, Jasminaroma, Lavendelaroma, Pfefferminzaroma, Himbeeraroma, Zitronenaroma, Kräuteraroma, Fleischgeschmack, Vanillin/Vanillearoma, Saccharin, Aspartam, Sorbit, Xylit;
(iv) Wasser, bevorzugt in Form von wässrigem Puffer, bevorzugter Citronensäurepuffer, noch bevorzugter wässrigem Puffer mit pH 4,5, am meisten bevorzugt Citronensäurepuffer mit pH 4,5;
(v) ein oder mehreren Lösungsvermittlern, bevorzugt ausgewählt aus der Gruppe bestehend aus: Tensiden, anionischen Tensiden, nichtionischen Tensiden, hydrierten Ricinusölen, Polyoxyethylen-Polyoxypropylen-Blockcopolymeren, Polyethylenglykolen, Propylenglykolderivaten, Polyvinylpyrrolidonen, bevorzugter ausgewählt aus der Gruppe bestehend aus: Natriumdodecylsulfat (SDS), Cremophor RH 40 (PEG-40 Hydrogenated Castor Oil, Macrogolglycerinhydroxystearat 40), Polysorbat 20, Lutrol F 68 (Poloxamer 188), PEG 200, PEG 300, PEG 400, Propylenglykolmonolaurat, Kollidon 12 (Povidon);
(vi) gegebenenfalls ein oder mehreren viskositätssteigernden Mitteln, bevorzugt ausgewählt aus der Gruppe bestehend aus: anorganischen Gelbildnern, organischen Gelbildnern, Cellulosederivaten, bevorzugter ausgewählt aus der Gruppe bestehend aus: Hydroxylethylcellulose, Hydroxylpropylmethylcellulose, Siliciumdioxid;
(vii) gegebenenfalls ein oder mehreren Antioxidationsmitteln, bevorzugt ausgewählt aus der Gruppe bestehend aus: Ascorbinsäure oder pharmazeutisch unbedenklichen Salzen davon, besonders Natriumascorbat; Citronensäure (wasserfrei und/oder Monohydrat) oder pharmazeutisch unbedenklichen Salzen davon, bevorzugter Natriumcitrat; Erythorbinsäure; Fumarsäure; Äpfelsäure; Monothioglycerin; Phosphorsäure; Natriumdisulfit; Kaliumdisulfit; Propionsäure; Natriumhydrogensulfit; Natriumsulfit; Resveratrol, Butylhydroxyanisol; Butylhydroxytoluol; Gallatderivaten, besonders Propylgallat, Octylgallat; Vitamin E oder pharmazeutisch unbedenklichen Salzen davon; Ascorbylpalmitat; Edetinsäure oder pharmazeutisch unbedenklichen Salzen davon; am meisten bevorzugt Natriumdisulfit.

15. Wässrige pharmazeutische Zusammensetzung nach einem der Ansprüche 1 bis 14, umfassend, vorzugsweise bestehend aus:
(i) 0,5 - 20,0 g/100 ml (0,5 - 20,0 % Gew./Vol.), bevorzugt 0,5 - 15,0 g/100 ml (0,5 - 15,0 % Gew./Vol.), bevorzugter 1,0 -1,5 g/100 ml (1,0 - 1,5 % Gew./Vol.) Velagliflozin als einziger SGLT-2-Inhibitor;
(ii) 0 - 3,0 g/100 ml (0 - 3,0 % Gew./Vol.), bevorzugt 0,05 - 3,0 g/100 ml (0,05 - 3,0 % Gew./Vol.), bevorzugter 0,05 - 2,0 g/100 ml (0,05 - 2,0 % Gew./Vol.) ein oder mehrere Konservierungsmittel; bevorzugter ausgewählt aus der Gruppe bestehend aus: Sorbinsäure oder Salzen davon, bevorzugt Natriumsorbat, Kaliumsorbat, Calciumsorbat; Benzoesäure oder Salzen davon, bevorzugt Natriumbenzoat; Benzalkoniumchlorid; Benzethoniumchlorid; Benzylalkohol, Cetylpyridiniumchlorid; Natriumdisulfit; Natriumacetat; Parabenen und Salze davon, bevorzugt Methylparaben, Ethylparaben, Propylparaben, Butylparaben, Butylparaben-Natrium; oder Kombinationen davon; am meisten bevorzugt Benzoesäure und/oder Salzen davon wie Natriumbenzoat;
(iii) 0 - 40 g/100 ml (0 - 40 % Gew./Vol.), bevorzugt 0 - 30 g/100 ml (0 - 30 % Gew./Vol.), bevorzugter 0 - 2 g/100 ml (0 - 2 % Gew./Vol.) ein oder mehrere Aromen und/oder Süßungsmittel, bevorzugter ausgewählt aus der Gruppe bestehend aus: Honigaroma, Limetten-/Salbeiaroma, Jasminaroma, Lavendelaroma, Pfefferminzaroma, Himbeeraroma, Zitronenaroma, Kräuteraroma, Fleischgeschmack, Vanillin/Vanillearoma, Saccharin, Aspartam, Sorbit, Xylit;
(iv) 30 bis 100 g/100 ml (30 bis 100 % Gew./Vol.), bevorzugt 30 bis 95 g/100 ml (30 bis 95 % Gew./Vol.), bevorzugter 30 bis 90 g/100 ml (30 bis 90 % Gew./Vol.), bevorzugter 30 bis 85 g/100 ml (30 bis 85 % Gew./Vol.), noch bevorzugter 30 bis 80 g/100 ml (30 bis 80 % Gew./Vol.), am meisten bevorzugt 50 bis 80 g/100 ml (50 bis 80 % Gew./Vol.) Wasser, bevorzugt in Form von wässrigem Puffer, bevorzugter Citronensäurepuffer, noch bevorzugter wässrigem Puffer mit pH 4,5, am meisten bevorzugt Citronensäurepuffer mit pH 4,5;
(v) 1 bis 50 g/100 ml (1 - 50 % Gew./Vol.), bevorzugt 1 bis 45 g/100 ml (1 - 45 % Gew./Vol.), noch bevorzugter 1 bis 40 g/100 ml (1-40 % Gew./Vol.), noch bevorzugter 1 bis 35 g/100 ml (1 - 35 % Gew./Vol.), noch bevorzugter 1 bis 30 g/100 ml (1 - 30 % Gew./Vol.), noch bevorzugter 1 bis 25 g/100 ml (1 - 25 % Gew./Vol.) am meisten bevorzugt 5 bis 25 g/100 ml (5 - 25 % Gew./Vol.) ein oder mehrere Lösungsvermittler, bevorzugt ausgewählt aus der Gruppe bestehend aus: Tensiden, anionischen Tensiden, nichtionischen Tensiden, hydrierten Ricinusölen, Polyoxyethylen-Polyoxypropylen-Blockcopolymeren, Polyethylenglykolen, Propylenglykolderivaten, Polyvinylpyrrolidonen, bevorzugter ausgewählt aus der Gruppe bestehend aus: Natriumdodecylsulfat (SDS), Cremophor RH 40 (PEG-40 Hydrogenated Castor Oil, Macrogolglycerinhydroxystearat 40), Polysorbat 20, Lutrol F 68 (Poloxamer 188), PEG 200, PEG 300, PEG 400, Propylenglykolmonolaurat, Kollidon 12 (Povidon);
(vi) 0 - 40 g/100 ml (0 - 40 % Gew./Vol.), bevorzugt 10 - 30 g/100 ml (10 - 30 % Gew./Vol.) ein oder mehrere viskositätssteigernde Mittel, bevorzugt ausgewählt aus der Gruppe bestehend aus: anorganischen Gelbildnern, organischen Gelbildnern, Cellulosederivaten, bevorzugter ausgewählt aus der Gruppe bestehend aus: Hydroxylethylcellulose, Hydroxylpropylmethylcellulose, Siliciumdioxid;
(vii) 0 - 1,0 g/100 ml (0 - 1,0 % Gew./Vol.), bevorzugt 0 - 0,5 g/100 ml (0 - 0,5 % Gew./Vol.), bevorzugter 0,1 - 0,3 g/100 ml (0,1 - 0,3 % Gew./Vol.) ein oder mehrere Antioxidationsmittel, bevorzugt ausgewählt aus der Gruppe bestehend aus: Ascorbinsäure oder pharmazeutisch unbedenklichen Salzen davon, besonders Natriumascorbat; Citronensäure (wasserfrei und/oder Monohydrat) oder pharmazeutisch unbedenklichen Salzen davon, bevorzugter Natriumcitrat; Erythorbinsäure; Fumarsäure; Äpfelsäure; Monothioglycerin; Phosphorsäure; Natriumdisulfit; Kaliumdisulfit; Propionsäure; Natriumhydrogensulfit; Natriumsulfit; Resveratrol, Butylhydroxyanisol; Butylhydroxytoluol; Gallatderivaten, besonders Propylgallat, Octylgallat; Vitamin E oder pharmazeutisch unbedenklichen Salzen davon; Ascorbylpalmitat; Edetinsäure oder pharmazeutisch unbedenklichen Salzen davon; am meisten bevorzugt Natriumdisulfit.

16. Wässrige pharmazeutische Zusammensetzung nach einem der Ansprüche 1 bis 15, umfassend, vorzugsweise bestehend aus:
Velagliflozin; Natriumdodecylsulfat (SDS), Cremophor RH 40 (PEG-40 Hydrogenated Castor Oil, Macrogolglycerinhydroxystearat 40), Polysorbat 20, Lutrol F 68 (Poloxamer 188), PEG 200, PEG 300, PEG 400, Propylenglykolmonolaurat und/oder Kollidon 12 (Povidon); Natriumbenzoat; gegebenenfalls Na-Disulfit; Wasser;
oder
Velagliflozin; Citronensäure-Monohydrat; NaOH; Natriumdodecylsulfat (SDS), Cremophor RH 40 (PEG-40 Hydrogenated Castor Oil, Macrogolglycerinhydroxystearat 40), Polysorbat 20, Lutrol F 68 (Poloxamer 188), PEG 200, PEG 300, PEG 400, Propylenglykolmonolaurat und/oder Kollidon 12 (Povidon); Natriumbenzoat; gegebenenfalls Na-Disulfit; Wasser;
oder
Velagliflozin; Citronensäure-Monohydrat; NaOH; Natriumdodecylsulfat (SDS), Cremophor RH 40 (PEG-40 Hydrogenated Castor Oil, Macrogolglycerinhydroxystearat 40), Polysorbat 20, Lutrol F 68 (Poloxamer 188), PEG 200, PEG 300, PEG 400, Propylenglykolmonolaurat und/oder Kollidon 12 (Povidon); Natriumbenzoat; gegebenenfalls Na-Disulfit; Sorbit und/oder Xylit; Honigaroma und/oder Vanillin und/oder Fleischgeschmack; Wasser.

17. Wässrige pharmazeutische Zusammensetzung nach einem der Ansprüche 1 bis 16, ausgewählt aus der Gruppe bestehend aus den folgenden Zusammensetzungen 5 bis 12:
| Bestandteil | Zusammensetzung 5 [mg/ml] | Zusammensetzung 6 [mg/ml] | Zusammensetzung 7 [mg/ml] | Zusammensetzung 8 [mg/ml] |
|---|---|---|---|---|
| Velagliflozin | 15,0 | 15,0 | 15,0 | 15,0 |
| Citronensäure-Monohydrat | 9,8 | 9,8 | 9,8 | 9,8 |
| NaOH 1 M | 46,4 | 46,4 | 46,4 | 46,4 |
| Kollidon 12 | - | 100,0 | 50,0 | - |
| PEG 300 | 150,0 | - | 100,0 | - |
| Natriumdodecyls ulfat (SDS; Na-Laurylsulfat) | - | - | - | 50,0 |
| Natriumbenzoat | 2,0 | 2,0 | 2,0 | 2,0 |
| Na-Disulfit | - | 2,0 | 2,0 | - |
| Sorbit | 170,0 | 170,0 | 170,0 | 170,0 |
| Honigaroma | 1,5 | 1,5 | 1,5 | 1,5 |
| Wasser, gereinigt | ad 1100 ml | ad 1100 ml | ad 1100 ml | ad 1100 ml |
| Bestandteil | Zusammensetzung 9 [mg/ml] | Zusammensetzung 10 [mg/ml] | Zusammensetzung 11 [mg/ml] | Zusammensetzung 12 [mg/ml] |
|---|---|---|---|---|
| Velagliflozin | 15,0 | 15,0 | 15,0 | 15,0 |
| Citronensäure-Monohydrat | 9,8 | 9,8 | 9,8 | 9,8 |
| NaOH 1 M | 46,4 | 46,4 | 46,4 | 46,4 |
| Cremophor RH40 | 250,0 | - | - | - |
| Polysorbat 80 | - | 250,0 | - | - |
| Poloxamer 188 | - | - | 50,0 | - |
| PEG 400 | - | - | - | 200,0 |
| Natriumbenzoat | 2,0 | 2,0 | 2,0 | 2,0 |
| Sorbit | 170,0 | 170,0 | 170,0 | 170,0 |
| Honigaroma | 1,5 | 1,5 | 1,5 | 1,5 |
| Wasser, gereinigt | ad 1100 ml | ad 1100 ml | ad 1100 ml | ad 1100 ml |

18. Wässrige pharmazeutische Zusammensetzung nach einem der Ansprüche 1 bis 17, wobei die wässrige pharmazeutische Zusammensetzung zur oralen und/oder parenteralen Verabreichung, vorzugsweise oralen Verabreichung bestimmt ist.

19. Wässrige pharmazeutische Zusammensetzung nach einem der Ansprüche 1 bis 18 zur Verwendung bei einem Verfahren zur Behandlung und/oder Vorbeugung einer oder mehrerer medizinischer Indikationen bei einem eine solche Behandlung und/oder Vorbeugung benötigenden Individuum, bevorzugt einem Tier, bevorzugter einem Säugetier, insbesondere einem Pferd, einer Katze, einem Hund oder einer Kuh, wobei die medizinischen Indikationen aus den folgenden ausgewählt sind:
(i) eine Stoffwechselstörung eines Equiden, wobei es sich vorzugsweise bei der Stoffwechselstörung um eine oder mehrere Störungen handelt, die ausgewählt sind aus Insulinresistenz, Hyperinsulinämie, beeinträchtigter Glucosetoleranz, Dyslipidämie, Dysadipokinämie, subklinischer Entzündung, systemischer Entzündung, niedriggradiger systemischer Entzündung, Obesität und/oder regionaler Adipositas, wobei die Stoffwechselstörung bevorzugt Insulinresistenz, Hyperinsulinämie und/oder ein klinisches Leiden ist, das mit Insulinresistenz und/oder Hyperinsulinämie assoziiert ist; wobei es sich vorzugsweise bei dem klinischen Leiden um ein oder mehrere Leiden handelt, die ausgewählt sind aus beeinträchtigter Glucosetoleranz, Dyslipidämie, Dysadipokinämie, subklinischer Entzündung, systemischer Entzündung, niedriggradiger systemischer Entzündung, Obesität und/oder regionaler Adipositas;
(ii) eine Stoffwechselstörung eines Equiden, wobei es sich bei der Stoffwechselstörung um eine oder mehrere Störungen handelt, die ausgewählt sind aus Laminitis, vaskulärer Dysfunktion, Hypertonie, hepatischer Lipidose, Atherosklerose, Hyperadrenokortizismus, PPID (Pituitary Pars Intermedia Dysfunction) und/oder EMS (Equine Metabolic Syndrome), wobei die Stoffwechselstörung bevorzugt ein klinisches Leiden/Zeichen ist, das mit Insulinresistenz und/oder Hyperinsulinämie assoziiert ist, wobei es sich vorzugsweise bei dem klinischen Leiden/Zeichen um ein oder mehrere Leiden handelt, die ausgewählt sind aus Laminitis, vaskulärer Dysfunktion, Hypertonie, hepatischer Lipidose, Atherosklerose, Hyperadrenokortizismus, PPID (Pituitary Pars Intermedia Dysfunction) und/oder EMS (Equine Metabolic Syndrome);
(iii) eine Stoffwechselstörung eines Feliden, wobei es sich vorzugsweise bei der Stoffwechselstörung um eine oder mehrere handelt, die ausgewählt sind aus der Gruppe bestehend aus: Ketoazidose, Prädiabetes, Diabetes mellitus Typ 1 oder Typ 2, Insulinresistenz, Akromegalie, Diabetes mit erhöhter IGF-1-Konzentration, Obesität, Hyperglykämie, beeinträchtigter Glucosetoleranz, Hyperinsulinämie, Dyslipidämie, Dysadipokinämie, subklinischer Entzündung, systemischer Entzündung, niedriggradiger systemischer Entzündung, hepatischer Lipidose, Atherosklerose, Entzündung der Bauchspeicheldrüse, Neuropathie und/oder Syndrom X (metabolisches Syndrom) und/oder Funktionsverlust pankreatischer Beta-Zellen und/oder wobei die Remission der Stoffwechselstörung, bevorzugt die diabetische Remission, erreicht und/oder aufrechterhalten wird;
(iv) eine Stoffwechselstörung eines Caniden, wobei es sich vorzugsweise bei der Stoffwechselstörung um eine oder mehrere handelt, die ausgewählt sind aus der Gruppe bestehend aus: Ketoazidose, Prädiabetes, insulinpflichtigem Diabetes mellitus, insulinresistentem Diabetes, Insulinresistenz, Obesität, Hyperglykämie, durch Hyperglykämie induzierter Kataraktbildung, beeinträchtigter Glucosetoleranz, Hyperinsulinämie, Dyslipidämie, Dysadipokinämie, subklinischer Entzündung, systemischer Entzündung, niedriggradiger systemischer Entzündung, hepatischer Lipidose, Entzündung der Bauchspeicheldrüse, Folgen von Stoffwechselstörungen, wie Hypertonie, Nierendysfunktion und/oder Muskel-Skelett-Erkrankungen, und/oder Syndrom X (metabolisches Syndrom), bevorzugt Prädiabetes, insulinpflichtigem Diabetes mellitus, insulinresistentem Diabetes, Insulinresistenz, wobei bevorzugt die Entwicklung von durch Hyperglykämie induzierter Kataraktbildung verhindert oder eine Remission erreicht wird und/oder wobei bevorzugt die Entwicklung von Folgen von Stoffwechselstörungen, wie Hypertonie, Nierendysfunktion und/oder Muskel-Skelett-Erkrankungen, verhindert oder die Progression verlangsamt oder eine Remission erreicht wird.
(v) eine Herzerkrankung eines Feliden, wobei es sich vorzugsweise bei der Herzerkrankung um eine oder mehrere handelt, die ausgewählt sind aus der Gruppe bestehend aus: Herzinsuffizienz, Herzinsuffizienz aufgrund einer oder mehrerer Kardiomyopathien, Herzinsuffizienz aufgrund hypertropher Kardiomyopathie (HCM), Herzinsuffizienz aufgrund restriktiver Kardiomyopathie (RCM), Herzinsuffizienz aufgrund dilatativer Kardiomyopathie (DCM), Herzinsuffizienz aufgrund nicht klassifizierter Kardiomyopathie (UCM), Herzinsuffizienz aufgrund arrhythmogener rechtsventrikulärer Kardiomyopathie (ARVC), hypertropher Kardiomyopathie (HCM), restriktiver Kardiomyopathie (RCM), dilatativer Kardiomyopathie (DCM), nicht klassifizierter Kardiomyopathie (UCM) und/oder arrhythmogener rechtsventrikulärer Kardiomyopathie (ARVC); vorzugsweise ausgewählt aus der Gruppe bestehend aus: Herzinsuffizienz aufgrund einer oder mehrerer Kardiomyopathien, Herzinsuffizienz aufgrund hypertropher Kardiomyopathie (HCM), hypertropher Kardiomyopathie (HCM);
(vi) Trockenstellen eines nichtmenschlichen Säugers, bevorzugt Wiederkäuers, Verbessern und/oder Erleichtern des Trockenstellens eines nichtmenschlichen Säugers, bevorzugt Wiederkäuers, Verringern der Milchproduktion, bevorzugt Milchproduktion und/oder -sekretion, bei einem trächtigen und/oder laktierenden nichtmenschlichen Säuger, bevorzugt Wiederkäuer, Verringern von Milchansammlung und/oder Milchstau im Euter, bevorzugt in Euter und/oder Milchdrüse, Vermindern der mit Eutermilchstau verbundenen Beschwerden, wie etwa Verlängern der täglichen Liegezeit und/oder Stressreduzierung, bei einem nichtmenschlichen Säuger, bevorzugt Wiederkäuer, Vermindern des Milchausflusses nach Trockenstellen eines nichtmenschlichen Säugers, bevorzugt Wiederkäuers, Vermindern der Inzidenz von intramammären Infektionen (IMI), bevorzugt Mastitis und/oder Metritis, bei einem nichtmenschlichen Säuger, bevorzugt Wiederkäuer;
(vii) eine Herzerkrankung eines nichtmenschlichen Säugers mit Ausnahme von Katzen, insbesondere eines Hundes, wobei es sich vorzugsweise bei der Herzerkrankung um eine oder mehrere handelt, die ausgewählt sind aus der Gruppe bestehend aus: Herzinsuffizienz; kongestiver Herzinsuffizienz; asymptomatischer / präklinischer / okkulter Herzinsuffizienz; Herzinsuffizienz aufgrund (myxomatöser) Mitralklappenerkrankung [(M)MVD]; kongestiver Herzinsuffizienz aufgrund (myxomatöser) Mitralklappenerkrankung [(M)MVD]; asymptomatischer / präklinischer / okkulter Herzinsuffizienz aufgrund (myxomatöser) Mitralklappenerkrankung [(M)MVD]; (myxomatöser) Mitralklappenerkrankung [(M)MVD]; Herzinsuffizienz aufgrund dilatativer Kardiomyopathie (DCM); kongestiver Herzinsuffizienz aufgrund dilatativer Kardiomyopathie (DCM); asymptomatischer / präklinischer / okkulter Herzinsuffizienz aufgrund dilatativer Kardiomyopathie (DCM); dilatativer Kardiomyopathie (DCM); klinisch manifester dilatativer Kardiomyopathie (DCM); asymptomatischer / präklinischer / okkulter dilatativer Kardiomyopathie (DCM); Aortenstenose (valvulär, supravalvulär und/oder subvalvulär);
(viii) Hypertonie bei einem nichtmenschlichen Säuger, bevorzugt einem Fleischfresser, bevorzugter einer Katze oder einem Hund, wobei es sich vorzugsweise bei der Hypertonie um eine oder mehrere handelt, die ausgewählt sind aus der Gruppe bestehend aus: situationsbedingter Hypertonie, sekundärer Hypertonie und idiopathischer Hypertonie, wobei die sekundäre Hypertonie vorzugsweise ausgewählt ist aus der Gruppe bestehend aus Hypertonie in Zusammenhang mit chronischer Nierenerkrankung (CNE), Diabetes, Obesität, Herzkrankheit, endokrinen Erkrankungen wie Cushing-Syndrom, Hyperthyreose, Akromegalie und durch Medikamente, bevorzugt durch Glucocorticoide, Mineralocorticoide, Erythropoese stimulierende Mittel, Ephedrin und/oder hochdosiertes Natriumchlorid, induziertem erhöhtem Blutdruck (BP);
(ix) eine Nierenerkrankung eines nichtmenschlichen Säugers, bevorzugt eines Fleischfressers, bevorzugter einer Katze oder eines Hundes, wobei es sich vorzugsweise bei der Nierenerkrankung um eine oder mehrere handelt, die ausgewählt sind aus der Gruppe bestehend aus: Nierendysplasie, Glomerulopathie, polyzystischer Nierenerkrankung, Amyloidose, Tubulo-Nephritis / tubulointerstitieller Nephritis (TIN), akuter Nierenerkrankung, chronischer Nierenerkrankung.

20. Verfahren zur Herstellung der wässrigen pharmazeutischen Zusammensetzung nach einem der Ansprüche 1 bis 18, umfassend (in sinnvoller Reihenfolge) die folgenden Schritte:
(i) Wasser wird als Ausgangsmaterial verwendet;
(ii) Zugeben ein oder mehrerer Lösungsvermittler zu Wasser, das aus Schritt (i) resultiert;
(iii) Lösen von Velagliflozin in dem aus Schritt (ii) resultierenden Gemisch;
(iv) gegebenenfalls Lösen der ein oder mehreren Konservierungsmittel in dem aus Schritt (iii) resultierenden Gemisch,
(v) gegebenenfalls weiteres Lösen weiterer Hilfsstoffe, wie etwa pH-Modifikator(en), Aromastoff(e), Süßungsmittel, Antioxidationsmittel, viskositätssteigernde Mittel und dergleichen, in dem aus Schritt (iii) oder gegebenenfalls (iv) resultierenden Gemisch;
(vi) gegebenenfalls Filtrieren des aus Schritt (iii), gegebenenfalls Schritt (iv) oder gegebenenfalls Schritt (v) resultierenden Gemischs;
wobei gegebenenfalls unabhängig voneinander nach jedem der einzelnen Verfahrensschritte - ob obligatorisch oder fakultativ - ein zusätzlicher Mischschritt durchgeführt wird.

21. Kit-of-parts, umfassend:
(a) eine wässrige pharmazeutische Zusammensetzung nach einem der Ansprüche 1 bis 18; und
(b) eine Packungsbeilage, die die Information enthält, dass die wässrige pharmazeutische Zusammensetzung zur Vorbeugung und/oder Behandlung einer oder mehrerer medizinischer Indikationen bei einem eine solche Vorbeugung und/oder Behandlung benötigenden Individuum zu verwenden ist, wobei die medizinischen Indikationen aus den folgenden ausgewählt sind:
(i) eine Stoffwechselstörung eines Equiden, wobei es sich vorzugsweise bei der Stoffwechselstörung um eine oder mehrere Störungen handelt, die ausgewählt sind aus Insulinresistenz, Hyperinsulinämie, beeinträchtigter Glucosetoleranz, Dyslipidämie, Dysadipokinämie, subklinischer Entzündung, systemischer Entzündung, niedriggradiger systemischer Entzündung, Obesität und/oder regionaler Adipositas, wobei die Stoffwechselstörung bevorzugt Insulinresistenz, Hyperinsulinämie und/oder ein klinisches Leiden ist, das mit Insulinresistenz und/oder Hyperinsulinämie assoziiert ist; wobei es sich vorzugsweise bei dem klinischen Leiden um ein oder mehrere Leiden handelt, die ausgewählt sind aus beeinträchtigter Glucosetoleranz, Dyslipidämie, Dysadipokinämie, subklinischer Entzündung, systemischer Entzündung, niedriggradiger systemischer Entzündung, Obesität und/oder regionaler Adipositas;
(ii) eine Stoffwechselstörung eines Equiden, wobei es sich bei der Stoffwechselstörung um eine oder mehrere Störungen handelt, die ausgewählt sind aus Laminitis, vaskulärer Dysfunktion, Hypertonie, hepatischer Lipidose, Atherosklerose, Hyperadrenokortizismus, PPID (Pituitary Pars Intermedia Dysfunction) und/oder EMS (Equine Metabolic Syndrome), wobei die Stoffwechselstörung bevorzugt ein klinisches Leiden/Zeichen ist, das mit Insulinresistenz und/oder Hyperinsulinämie assoziiert ist, wobei es sich vorzugsweise bei dem klinischen Leiden/Zeichen um ein oder mehrere Leiden handelt, die ausgewählt sind aus Laminitis, vaskulärer Dysfunktion, Hypertonie, hepatischer Lipidose, Atherosklerose, Hyperadrenokortizismus, PPID (Pituitary Pars Intermedia Dysfunction) und/oder EMS (Equine Metabolic Syndrome);
(iii) eine Stoffwechselstörung eines Feliden, wobei es sich vorzugsweise bei der Stoffwechselstörung um eine oder mehrere handelt, die ausgewählt sind aus der Gruppe bestehend aus: Ketoazidose, Prädiabetes, Diabetes mellitus Typ 1 oder Typ 2, Insulinresistenz, Akromegalie, Diabetes mit erhöhter IGF-1-Konzentration, Obesität, Hyperglykämie, beeinträchtigter Glucosetoleranz, Hyperinsulinämie, Dyslipidämie, Dysadipokinämie, subklinischer Entzündung, systemischer Entzündung, niedriggradiger systemischer Entzündung, hepatischer Lipidose, Atherosklerose, Entzündung der Bauchspeicheldrüse, Neuropathie und/oder Syndrom X (metabolisches Syndrom) und/oder Funktionsverlust pankreatischer Beta-Zellen und/oder wobei die Remission der Stoffwechselstörung, bevorzugt die diabetische Remission, erreicht und/oder aufrechterhalten wird;
(iv) eine Stoffwechselstörung eines Caniden, wobei es sich vorzugsweise bei der Stoffwechselstörung um eine oder mehrere handelt, die ausgewählt sind aus der Gruppe bestehend aus: Ketoazidose, Prädiabetes, insulinpflichtigem Diabetes mellitus, insulinresistentem Diabetes, Insulinresistenz, Obesität, Hyperglykämie, durch Hyperglykämie induzierter Kataraktbildung, beeinträchtigter Glucosetoleranz, Hyperinsulinämie, Dyslipidämie, Dysadipokinämie, subklinischer Entzündung, systemischer Entzündung, niedriggradiger systemischer Entzündung, hepatischer Lipidose, Entzündung der Bauchspeicheldrüse, Folgen von Stoffwechselstörungen, wie Hypertonie, Nierendysfunktion und/oder Muskel-Skelett-Erkrankungen, und/oder Syndrom X (metabolisches Syndrom), bevorzugt Prädiabetes, insulinpflichtigem Diabetes mellitus, insulinresistentem Diabetes, Insulinresistenz, wobei bevorzugt die Entwicklung von durch Hyperglykämie induzierter Kataraktbildung verhindert oder eine Remission erreicht wird und/oder wobei bevorzugt die Entwicklung von Folgen von Stoffwechselstörungen, wie Hypertonie, Nierendysfunktion und/oder Muskel-Skelett-Erkrankungen, verhindert oder die Progression verlangsamt oder eine Remission erreicht wird.
(v) eine Herzerkrankung eines Feliden, wobei es sich vorzugsweise bei der Herzerkrankung um eine oder mehrere handelt, die ausgewählt sind aus der Gruppe bestehend aus: Herzinsuffizienz, Herzinsuffizienz aufgrund einer oder mehrerer Kardiomyopathien, Herzinsuffizienz aufgrund hypertropher Kardiomyopathie (HCM), Herzinsuffizienz aufgrund restriktiver Kardiomyopathie (RCM), Herzinsuffizienz aufgrund dilatativer Kardiomyopathie (DCM), Herzinsuffizienz aufgrund nicht klassifizierter Kardiomyopathie (UCM), Herzinsuffizienz aufgrund arrhythmogener rechtsventrikulärer Kardiomyopathie (ARVC), hypertropher Kardiomyopathie (HCM), restriktiver Kardiomyopathie (RCM), dilatativer Kardiomyopathie (DCM), nicht klassifizierter Kardiomyopathie (UCM) und/oder arrhythmogener rechtsventrikulärer Kardiomyopathie (ARVC); vorzugsweise ausgewählt aus der Gruppe bestehend aus: Herzinsuffizienz aufgrund einer oder mehrerer Kardiomyopathien, Herzinsuffizienz aufgrund hypertropher Kardiomyopathie (HCM), hypertropher Kardiomyopathie (HCM);
(vi) Trockenstellen eines nichtmenschlichen Säugers, bevorzugt Wiederkäuers, Verbessern und/oder Erleichtern des Trockenstellens eines nichtmenschlichen Säugers, bevorzugt Wiederkäuers, Verringern der Milchproduktion, bevorzugt Milchproduktion und/oder -sekretion, bei einem trächtigen und/oder laktierenden nichtmenschlichen Säuger, bevorzugt Wiederkäuer, Verringern von Milchansammlung und/oder Milchstau im Euter, bevorzugt in Euter und/oder Milchdrüse, Vermindern der mit Eutermilchstau verbundenen Beschwerden, wie etwa Verlängern der täglichen Liegezeit und/oder Stressreduzierung, bei einem nichtmenschlichen Säuger, bevorzugt Wiederkäuer, Vermindern des Milchausflusses nach Trockenstellen eines nichtmenschlichen Säugers, bevorzugt Wiederkäuers, Vermindern der Inzidenz von intramammären Infektionen (IMI), bevorzugt Mastitis und/oder Metritis, bei einem nichtmenschlichen Säuger, bevorzugt Wiederkäuer;
(vii) eine Herzerkrankung eines nichtmenschlichen Säugers mit Ausnahme von Katzen, insbesondere eines Hundes, wobei es sich vorzugsweise bei der Herzerkrankung um eine oder mehrere handelt, die ausgewählt sind aus der Gruppe bestehend aus: Herzinsuffizienz; kongestiver Herzinsuffizienz; asymptomatischer / präklinischer / okkulter Herzinsuffizienz; Herzinsuffizienz aufgrund (myxomatöser) Mitralklappenerkrankung [(M)MVD]; kongestiver Herzinsuffizienz aufgrund (myxomatöser) Mitralklappenerkrankung [(M)MVD]; asymptomatischer / präklinischer / okkulter Herzinsuffizienz aufgrund (myxomatöser) Mitralklappenerkrankung [(M)MVD]; (myxomatöser) Mitralklappenerkrankung [(M)MVD]; Herzinsuffizienz aufgrund dilatativer Kardiomyopathie (DCM); kongestiver Herzinsuffizienz aufgrund dilatativer Kardiomyopathie (DCM); asymptomatischer / präklinischer / okkulter Herzinsuffizienz aufgrund dilatativer Kardiomyopathie (DCM); dilatativer Kardiomyopathie (DCM); klinisch manifester dilatativer Kardiomyopathie (DCM); asymptomatischer / präklinischer / okkulter dilatativer Kardiomyopathie (DCM); Aortenstenose (valvulär, supravalvulär und/oder subvalvulär);
(viii) Hypertonie bei einem nichtmenschlichen Säuger, bevorzugt einem Fleischfresser, bevorzugter einer Katze oder einem Hund, wobei es sich vorzugsweise bei der Hypertonie um eine oder mehrere handelt, die ausgewählt sind aus der Gruppe bestehend aus: situationsbedingter Hypertonie, sekundärer Hypertonie und idiopathischer Hypertonie, wobei die sekundäre Hypertonie vorzugsweise ausgewählt ist aus der Gruppe bestehend aus Hypertonie in Zusammenhang mit chronischer Nierenerkrankung (CNE), Diabetes, Obesität, Herzkrankheit, endokrinen Erkrankungen wie Cushing-Syndrom, Hyperthyreose, Akromegalie und durch Medikamente, bevorzugt durch Glucocorticoide, Mineralocorticoide, Erythropoese stimulierende Mittel, Ephedrin und/oder hochdosiertes Natriumchlorid, induziertem erhöhtem Blutdruck (BP);
(ix) eine Nierenerkrankung eines nichtmenschlichen Säugers, bevorzugt eines Fleischfressers, bevorzugter einer Katze oder eines Hundes, wobei es sich vorzugsweise bei der Nierenerkrankung um eine oder mehrere handelt, die ausgewählt sind aus der Gruppe bestehend aus: Nierendysplasie, Glomerulopathie, polyzystischer Nierenerkrankung, Amyloidose, Tubulo-Nephritis / tubulointerstitieller Nephritis (TIN), akuter Nierenerkrankung, chronischer Nierenerkrankung.

## Revendications

1. Composition pharmaceutique aqueuse contenant au moins un inhibiteur SGLT-2 et un ou plusieurs agents solubilisants, dans laquelle :
(i) l'au moins un inhibiteur SGLT-2 est la vélagliflozine,
(ii) la vélagliflozine est le seul inhibiteur SGLT-2 contenu dans une telle composition pharmaceutique aqueuse,
(iii) une telle composition pharmaceutique aqueuse est sensiblement exempte de solvants organiques, de préférence ne contient aucun solvant organique, et
(iv) le ou les agents solubilisants sont choisis dans le groupe constitué par : tensioactifs, tensioactifs anioniques, tensioactifs non ioniques, huiles de ricin hydrogénées, copolymères séquencés polyoxyéthylène-polyoxypropylène, polyéthylène glycols, dérivés de propylène glycol, polyvinylpyrrolidones, de préférence choisis dans le groupe constitué par : dodécylsulfate de sodium (SDS), Cremophor RH 40 (huile de ricin hydrogénée PEG-40, hydroxystéarate de macrogol glycérol 40), polysorbate 20, Lutrol F 68 (poloxamère 188), PEG 200, PEG 300, PEG 400, monolaurate de propylène glycol, Kollidon 12 (povidone).

2. Composition pharmaceutique aqueuse selon la revendication 1, la composition pharmaceutique aqueuse étant pour une administration, de préférence directe, à un sujet, de préférence sans étapes supplémentaires de traitement et/ou de purification obligatoires, plus préférablement un animal, encore plus préférablement un mammifère, en particulier un cheval, un chat, un chien ou une vache et/ou dans laquelle la composition pharmaceutique aqueuse est stérile.

3. Composition pharmaceutique aqueuse selon l'une quelconque des revendications 1 à 2, dans laquelle la quantité totale du ou des agents solubilisants est de 1 à 50 g/100 mL (1 à 50 % p/v), plus préférablement de 1 à 45 g/100 mL (1 à 45 % p/v), encore plus préférablement de 1 à 40 g/100 mL (1 à 40 % p/v), encore plus préférablement de 1 à 35 g/100 mL (1 à 35 % p/v), encore plus préférablement de 1 à 30 g/100 mL (1 à 30 % p/v), encore plus préférablement de 1 à 25 g/100 mL (1 à 25 % p/v), le plus préférablement de 5 à 25 g/100 mL (5 à 25 % p/v).

4. Composition pharmaceutique aqueuse selon l'une quelconque des revendications 1 à 3, dans laquelle deux ou plusieurs agents solubilisants sont contenus dans la composition pharmaceutique aqueuse.

5. Composition pharmaceutique aqueuse selon la revendication 4, dans laquelle deux agents solubilisants sont contenus dans une telle composition pharmaceutique aqueuse, de préférence la quantité du premier agent solubilisant et la quantité du deuxième agent solubilisant étant indépendamment l'une de l'autre choisies de 1 à 50 g/100 mL (1 à 50 % p/v), plus préférablement de 1 à 45 g/100 mL (1 à 45 % p/v), encore plus préférablement de 1 à 40 g/100 mL (1 à 40 % p/v), encore plus préférablement de 1 à 35 g/100 mL (1 à 35 % p/v), encore plus préférablement de 1 à 30 g/100 mL (1 à 30 % p/v), encore plus préférablement de 1 à 25 g/100 mL (1 à 25 % p/v), le plus préférablement de 5 à 25 g/100 mL (5 à 25 % p/v).

6. Composition pharmaceutique aqueuse selon la revendication 5, dans laquelle les deux agents solubilisants sont indépendamment l'un de l'autre choisis dans le groupe constitué par : dodécylsulfate de sodium (SDS), Cremophor RH 40 (huile de ricin hydrogénée PEG-40, hydroxystéarate de macrogol glycérol 40), polysorbate 20, Lutrol F 68 (poloxamère 188), PEG 200, PEG 300, PEG 400, monolaurate de propylène glycol, Kollidon 12 (povidone), de préférence dans laquelle ces deux agents solubilisants sont Kollidon 12 (povidone) ainsi que PEG 200, PEG 300 ou PEG 400, plus préférablement dans laquelle ces deux agents solubilisants sont Kollidon 12 (povidone) et PEG 300.

7. Composition pharmaceutique aqueuse selon l'une quelconque des revendications 1 à 6, dans laquelle la composition pharmaceutique aqueuse est une solution, une émulsion ou une suspension, de préférence une solution, une émulsion ou une suspension ayant une valeur NTU égale ou inférieure à 10,0, plus préférablement égale ou inférieure à 7,0, encore plus préférablement égale ou inférieure à 3,0, dans laquelle, de préférence, la composition pharmaceutique aqueuse est une solution ayant une valeur NTU égale ou inférieure à 3,0.

8. Composition pharmaceutique aqueuse selon l'une quelconque des revendications 1 à 7, dans laquelle une telle composition pharmaceutique aqueuse contient de 30 à 100 g/100 mL (30 à 100 % p/v), de préférence de 30 à 95 g/100 mL (30 à 95 % p/v), plus préférablement de 30 à 90 g/100 mL (30 à 90 % p/v), plus préférablement de 30 à 85 g/100 mL (30 à 85 % p/v), encore plus préférablement de 30 à 80 g/100 mL (30 à 80 % p/v), le plus préférablement de 50 à 80 g/100 mL d'eau (50 à 80 % p/v), plus préférablement sous la forme d'un tampon aqueux, tel qu'un tampon acide citrique.

9. Composition pharmaceutique aqueuse selon la revendication 8, dans laquelle une telle composition pharmaceutique aqueuse a une valeur de pH allant de 2 à 7, de préférence de 3 à 7, plus préférablement de 3,0 à 6,5, encore plus préférablement de 4,0 à 6,5, encore plus préférablement de 4,0 à 5,0, et le plus préférablement de 4,5.

10. Composition pharmaceutique aqueuse selon l'une quelconque des revendications 1 à 9, dans laquelle une telle composition pharmaceutique aqueuse comprend en outre un ou plusieurs conservateurs, de préférence choisis dans le groupe constitué par : acide sorbique ou des sels correspondants, plus préférablement le sorbate de sodium, le sorbate de potassium, le sorbate de calcium ; acide benzoïque ou des sels correspondants, plus préférablement le benzoate de sodium ; chlorure de benzalkonium ; chlorure de benzéthonium ; alcool benzylique, chlorure de cétylpyridinium ; métabisulfite de sodium ; acétate de sodium ; parabènes et des sels correspondants, plus préférablement le méthylparabène, l'éthylparabène, le propylparabène, le butylparabène, le butylparabène sodique ; et le plus préférablement l'acide benzoïque et/ou des sels correspondants, tels que le benzoate de sodium.

11. Composition pharmaceutique aqueuse selon l'une quelconque des revendications 1 à 10, dans laquelle une telle composition pharmaceutique aqueuse comprend de plus un ou plusieurs agents anti-oxydation, de préférence choisis dans le groupe constitué par : acide ascorbique ou des sels pharmaceutiquement acceptables correspondants, notamment l'ascorbate de sodium ; acide citrique (anhydre et/ou monohydraté) ou des sels pharmaceutiquement acceptables correspondants, plus préférablement le citrate de sodium ; acide érythorbique ; acide fumarique ; acide malique ; monothioglycérol ; acide phosphorique ; métabisulfite de sodium ; métabisulfite de potassium ; acide propionique ; bisulfite de sodium ; sulfite de sodium ; resvératrol ; butylhydroxyanisole ; butylhydroxytoluène ; dérivés de gallate, notamment le gallate de propyle et le gallate d'octyle ; vitamine E ou des sels pharmaceutiquement acceptables correspondants ; palmitate d'ascorbyle ; acide édétique ou des sels pharmaceutiquement acceptables correspondants ; le plus préférablement le métabisulfite de sodium.

12. Composition pharmaceutique aqueuse selon l'une quelconque des revendications 1 à 11, dans laquelle une telle composition pharmaceutique aqueuse comprend en outre un ou plusieurs agents d'amélioration de la viscosité, de préférence choisis dans le groupe constitué par : les agents inorganiques de formation de gel, les agents organiques de formation de gel, les dérivés de cellulose, plus préférablement choisis dans le groupe constitué par : l'hydroxyéthylcellulose, l'hydroxypropylméthylcellulose, le dioxyde de silicium.

13. Composition pharmaceutique aqueuse selon l'une quelconque des revendications 1 à 12, dans laquelle une telle composition pharmaceutique aqueuse comprend de plus un ou plusieurs arômes et/ou édulcorants, de préférence choisis dans le groupe constitué par : arôme de miel, arôme de citron vert/salvia, arôme de jasmin, arôme de lavande, arôme de menthe poivrée, arôme de framboise, arôme de citron, arôme d'herbes, arôme de viande, arôme de vanilline/vanille, saccharine, aspartame, sorbitol, xylitol.

14. Composition pharmaceutique aqueuse selon l'une quelconque des revendications 1 à 13 contenant, de préférence constituée de :
(i) vélagliflozine en tant qu'inhibiteur SGLT-2 unique ;
(ii) éventuellement, mais préférablement, un ou plusieurs conservateurs ; de préférence choisi dans le groupe constitué par : acide ascorbique ou des sels correspondants, de préférence sorbate de sodium, sorbate de potassium, sorbate de calcium ; acide benzoïque ou des sels correspondants, de préférence benzoate de sodium ; chlorure de benzalkonium ; chlorure de benzéthonium ; alcool benzylique, chlorure de cétylpyridinium ; métabisulfite de sodium ; acétate de sodium ; parabènes et des sels correspondants, de préférence méthylparabène, éthylparabène, propylparabène, butylparabène, butylparabène sodique ; ou des combinaisons correspondantes ; de préférence l'acide benzoïque et/ou des sels correspondants, tels que le benzoate de sodium ;
(iii) éventuellement, un ou plusieurs arômes et/ou édulcorants, de préférence choisis dans le groupe constitué par : arôme de miel, arôme de citron vert/salvia, arôme de jasmin, arôme de lavande, arôme de menthe poivrée, arôme de framboise, arôme de citron, arôme d'herbes, arôme de viande, arôme de vanilline/vanille, saccharine, aspartame, sorbitol, xylitol ;
(iv) de l'eau, de préférence sous forme de tampon aqueux, plus préférablement un tampon acide citrique, encore plus préférablement un tampon aqueux présentant un pH de 4,5, le plus préférablement un tampon acide citrique ayant un pH de 4,5 ;
(v) un ou plusieurs agents solubilisants, de préférence choisis dans le groupe constitué par : les tensioactifs, les tensioactifs anioniques, les tensioactifs non ioniques, les huiles de ricin hydrogénées, les copolymères séquencés de polyoxyéthylène-polyoxypropylène, les polyéthylène glycols, les dérivés de propylène glycol, les polyvinylpyrrolidones, plus préférablement choisis dans le groupe constitué par : dodécylsulfate de sodium (SDS), Cremophor RH 40 (huile de ricin hydrogénée PEG-40, hydroxystéarate de macrogol glycérol 40), polysorbate 20, Lutrol F 68 (poloxamère 188), PEG 200, PEG 300, PEG 400, monolaurate de propylène glycol, Kollidon 12 (povidone) ;
(vi) éventuellement, un ou plusieurs agents d'amélioration de la viscosité, préférablement choisis dans le groupe constitué par : les agents inorganiques de formation de gel, les agents organiques de formation de gel, les dérivés de cellulose, plus préférablement choisis dans le groupe constitué par : l'hydroxyéthylcellulose, l'hydroxypropylméthylcellulose, le dioxyde de silicium ;
(vii) éventuellement un ou plusieurs agents anti-oxydation, de préférence choisis dans le groupe constitué par : acide ascorbique ou des sels pharmaceutiquement acceptables correspondants, notamment l'ascorbate de sodium ; acide citrique (anhydre et/ou monohydraté) ou des sels pharmaceutiquement acceptables correspondants, plus préférablement le citrate de sodium ; acide érythorbique ; acide fumarique ; acide malique ; monothioglycérol ; acide phosphorique ; métabisulfite de sodium ; métabisulfite de potassium ; acide propionique ; bisulfite de sodium ; sulfite de sodium ; resvératrol ; butylhydroxyanisole ; butylhydroxytoluène ; dérivés de gallate, notamment le gallate de propyle et le gallate d'octyle ; vitamine E ou des sels pharmaceutiquement acceptables correspondants ; palmitate d'ascorbyle ; acide édétique ou des sels pharmaceutiquement acceptables correspondants ; le plus préférablement le métabisulfite de sodium.

15. Composition pharmaceutique aqueuse selon l'une quelconque des revendications 1 à 14 contenant, de préférence constituée de :
(i) 0,5 à 20,0 g/100 mL (0,5 à 20,0 % p/v), de préférence 0,5 à 15,0 g/100 mL (0,5 à 15,0 % p/v), plus préférablement 1,0 à 1,5 g/100 mL (1,0 à 1,5 % p/v) de vélagliflozine en tant qu'inhibiteur SGLT-2 unique ;
(ii) 0 à 3,0 g/100 mL (0 à 3,0 % p/v), de préférence 0,05 à 3,0 g/100 mL (0,05 à 3,0 % p/v), plus préférablement 0,05 à 2,0 g/100 mL (0,05 à 2,0 % p/v) d'un ou plusieurs conservateurs ; plus préférablement choisis dans le groupe constitué par : acide sorbique ou des sels correspondants, de préférence sorbate de sodium, sorbate de potassium, sorbate de calcium ; acide benzoïque ou des sels correspondants, de préférence benzoate de sodium ; chlorure de benzalkonium ; chlorure de benzéthonium ; alcool benzylique, chlorure de cétylpyridinium ; métabisulfite de sodium ; acétate de sodium ; parabènes et des sels correspondants, de préférence méthylparabène, éthylparabène, propylparabène, butylparabène, butylparabène sodique ; ou des combinaisons correspondantes ; le plus préférablement acide benzoïque et/ou des sels correspondants, tels que le benzoate de sodium ;
(iii) 0 à 40 g/100 mL (0 à 40 % p/v), de préférence 0 à 30 g/100 mL (0 à 30 % p/v), plus préférablement 0 à 2 g/100 mL (0 à 2 % p/v) d'un ou plusieurs arômes et/ou édulcorants, plus préférablement choisis dans le groupe constitué par : arôme de miel, arôme de citron vert/salvia, arôme de jasmin, arôme de lavande, arôme de menthe poivrée, arôme de framboise, arôme de citron, arôme d'herbes, arôme de viande, arôme de vanilline/vanille, saccharine, aspartame, sorbitol, xylitol ;
(iv) 30 à 100 g/100 mL (30 à 100 % p/v), préférablement de 30 à 95 g/100 mL (30 à 95 % p/v), plus préférablement de 30 à 90 g/100 mL (30 à 90 % p/v), plus préférablement de 30 à 85 g/100 mL (30 à 85 % p/v), encore plus préférablement de 30 à 80 g/100 mL (30 à 80 % p/v), le plus préférablement de 50 à 80 g/100 mL (50 à 80 % p/v) d'eau, de préférence sous la forme d'un tampon aqueux, plus préférablement d'un tampon acide citrique, encore plus préférablement d'un tampon aqueux ayant un pH de 4,5, le plus préférablement, d'un tampon acide citrique ayant un pH de 4,5 ;
(v) 1 à 50 g/100 mL (1 à 50 % p/v), préférablement de 1 à 45 g/100 mL (1 à 45 % p/v), encore plus préférablement de 1 à 40 g/100 mL (1 à 40 % p/v), encore plus préférablement de 1 à 35 g/100 mL (1 à 35 % p/v), encore plus préférablement de 1 à 30 g/100 mL (1 à 30 % p/v), encore plus préférablement de 1 à 25 g/100 mL (1 à 25 % p/v), le plus préférablement de 5 à 25 g/100 mL (5 à 25 % p/v) d'un ou plusieurs agents solubilisants, de préférence choisis dans le groupe constitué par : tensioactifs, tensioactifs anioniques, tensioactifs non ioniques, huiles de ricin hydrogénées, copolymères séquencés de polyoxyéthylène-polyoxypropylène, polyéthylène glycols, dérivés de propylène glycol, polyvinylpyrrolidones, plus préférablement choisis dans le groupe constitué par : dodécylsulfate de sodium (SDS), Cremophor RH 40 (huile de ricin hydrogénée PEG-40, hydroxystéarate de macrogol glycérol 40), polysorbate 20, Lutrol F 68 (poloxamère 188), PEG 200, PEG 300, PEG 400, monolaurate de propylène glycol, Kollidon 12 (povidone) ;
(vi) 0 à 40 g/100 mL (0 à 40 % p/v), préférablement 10 à 30 g/100 mL (10 à 30 % p/v) d'un ou plusieurs agents d'amélioration de la viscosité, préférablement choisis dans le groupe constitué par : les agents inorganiques de formation de gel, les agents organiques de formation de gel, les dérivés de cellulose, plus préférablement choisis dans le groupe constitué par : l'hydroxyéthylcellulose, l'hydroxypropylméthylcellulose, le dioxyde de silicium ;
(vii) 0 à 1,0 g/100 mL (0 à 1,0 % p/v), de préférence 0 à 0,5 g/100 mL (0 à 0,5 % p/v), plus préférablement 0,1 à 0,3 g/100 mL (0,1 à 0,3 % p/v) d'un ou plusieurs agents antioxydation, de préférence choisis dans le groupe constitué par : acide ascorbique ou des sels pharmaceutiquement acceptables correspondants, notamment l'ascorbate de sodium ; acide citrique (anhydre et/ou monohydraté) ou des sels pharmaceutiquement acceptables correspondants, plus préférablement le citrate de sodium ; acide érythorbique ; acide fumarique ; acide malique ; monothioglycérol ; acide phosphorique ; métabisulfite de sodium ; métabisulfite de potassium ; acide propionique ; bisulfite de sodium ; sulfite de sodium ; resvératrol ; butylhydroxyanisole ; butylhydroxytoluène ; dérivés de gallate, notamment le gallate de propyle et le gallate d'octyle ; vitamine E ou des sels pharmaceutiquement acceptables correspondants ; palmitate d'ascorbyle ; acide édétique ou des sels pharmaceutiquement acceptables correspondants ; le plus préférablement le métabisulfite de sodium.

16. Composition pharmaceutique aqueuse selon l'une quelconque des revendications 1 à 15 contenant, de préférence constituée de :
vélagliflozine ; dodécylsulfate de sodium (SDS), Cremophor RH 40 (huile de ricin hydrogénée PEG-40, hydroxystéarate de macrogol glycérol 40), polysorbate 20, Lutrol F 68 (poloxamère 188), PEG 200, PEG 300, PEG 400, monolaurate de propylène glycol et/ou Kollidon 12 (povidone) ; benzoate de sodium ; éventuellement métabisulfite de Na ; eau ;
ou
vélagliflozine ; monohydrate d'acide citrique ; NaOH ; dodécylsulfate de sodium (SDS), Cremophor RH 40 (huile de ricin hydrogénée PEG-40, hydroxystéarate de macrogol glycérol 40), polysorbate 20, Lutrol F 68 (poloxamère 188), PEG 200, PEG 300, PEG 400, monolaurate de propylène glycol et/ou Kollidon 12 (povidone) ; benzoate de sodium ; éventuellement métabisulfite de Na ; eau ;
ou
vélagliflozine ; monohydrate d'acide citrique ; NaOH ; dodécylsulfate de sodium (SDS), Cremophor RH 40 (huile de ricin hydrogénée PEG-40, hydroxystéarate de macrogol glycérol 40), polysorbate 20, Lutrol F 68 (poloxamère 188), PEG 200, PEG 300, PEG 400, monolaurate de propylène glycol et/ou Kollidon 12 (povidone) ; benzoate de sodium ; éventuellement, métabisulfite de Na ; sorbitol et/ou xylitol ; arôme de miel et/ou vanilline et/ou arôme de viande ; eau.

17. Composition pharmaceutique aqueuse selon l'une quelconque des revendications 1 à 16 choisie dans le groupe constitué par les compositions 5 à 12 suivantes :
| Ingrédient | Composition 5 [mg/mL] | Composition 6 [mg/mL] | Composition 7 [mg/mL] | Composition 8 [mg/mL] |
|---|---|---|---|---|
| Vélagliflozine | 15,0 | 15,0 | 15,0 | 15,0 |
| Monohydrate d'acide citrique | 9,8 | 9,8 | 9,8 | 9,8 |
| NaOH 1 M | 46,4 | 46,4 | 46,4 | 46,4 |
| Kollidon 12 | - | 100,0 | 50,0 | - |
| PEG 300 | 150,0 | - | 100,0 | - |
| Dodécylsulfate de sodium (SDS ; Laurylsulfate de Na) | - | - | - | 50,0 |
| Benzoate de sodium | 2,0 | 2,0 | 2,0 | 2,0 |
| Métabisulfite de Na | - | 2,0 | 2,0 | - |
| Sorbitol | 170,0 | 170,0 | 170,0 | 170,0 |
| Arôme de miel | 1,5 | 1,5 | 1,5 | 1,5 |
| Eau, purifiée | ad. 1 100 mL | ad. 1 100 mL | ad. 1 100 mL | ad. 1 100 mL |
| Ingrédient | Composition 9 [mg/mL] | Composition 10 [mg/mL] | Composition 11 [mg/mL] | Composition 12 [mg/mL] |
|---|---|---|---|---|
| Vélagliflozine | 15,0 | 15,0 | 15,0 | 15,0 |
| Monohydrate d'acide citrique | 9,8 | 9,8 | 9,8 | 9,8 |
| NaOH 1 M | 46,4 | 46,4 | 46,4 | 46,4 |
| Cremophor RH40 | 250,0 | - | - | - |
| Polysorbate 80 | - | 250,0 | - | - |
| Poloxamère 188 | - | - | 50,0 | - |
| PEG 400 | - | - | - | 200,0 |
| Benzoate de sodium | 2,0 | 2,0 | 2,0 | 2,0 |
| Sorbitol | 170,0 | 170,0 | 170,0 | 170,0 |
| Arôme de miel | 1,5 | 1,5 | 1,5 | 1,5 |
| Eau, purifiée | ad. 1 100 mL | ad. 1 100 mL | ad. 1 100 mL | ad. 1 100 mL |

18. Composition pharmaceutique aqueuse selon l'une quelconque des revendications 1 à 17, dans laquelle une telle composition pharmaceutique aqueuse est destinée à une administration orale et/ou parentérale, de préférence à une administration orale.

19. Composition pharmaceutique aqueuse selon l'une quelconque des revendications 1 à 18 pour utilisation dans un procédé de traitement et/ou de prévention d'une ou plusieurs indications médicales chez un sujet nécessitant un tel traitement et/ou une telle prévention, de préférence un animal, plus préférablement un mammifère, en particulier un cheval, un chat, un chien ou une vache, choisie parmi les indications médicales :
(i) un trouble métabolique d'un équidé, de préférence dans laquelle le trouble métabolique est un ou plusieurs troubles choisis parmi la résistance à l'insuline, l'hyperinsulinémie, l'intolérance au glucose, la dyslipidémie, la dysadipokinémie, l'inflammation subclinique, l'inflammation systémique, l'inflammation systémique de bas grade, l'obésité et/ou l'adiposité régionale, de préférence une résistance à l'insuline, une hyperinsulinémie et/ou une affection clinique associée à la résistance à l'insuline et/ou à l'hyperinsulinémie ; de préférence ladite affection clinique étant une ou plusieurs affections choisies parmi l'intolérance au glucose, la dyslipidémie, la dysadipokinémie, l'inflammation subclinique, l'inflammation systémique, l'inflammation systémique de bas grade, l'obésité et/ou l'adiposité régionale ;
(ii) un trouble métabolique d'un équidé, dans laquelle le trouble métabolique est un ou plusieurs troubles choisis parmi la fourbure, le dysfonctionnement vasculaire, l'hypertension, la lipidose hépatique, l'athérosclérose, l'hyperadrénocorticisme, le dysfonctionnement de la pars intermedia de l'hypophyse et/ou le syndrome métabolique équin, de préférence le trouble métabolique étant un état/signe clinique associé à une résistance à l'insuline et/ou à une hyperinsulinémie, ledit état/signe clinique étant de préférence un ou plusieurs états choisis parmi la fourbure, le dysfonctionnement vasculaire, l'hypertension, la lipidose hépatique, l'athérosclérose, l'hyperadrénocorticisme, le dysfonctionnement de la pars intermedia de l'hypophyse et/ou le syndrome métabolique équin ;
(iii) un trouble métabolique d'un animal félin, dans laquelle de préférence le trouble métabolique est l'un ou plusieurs choisis dans le groupe constitué par : acidocétose, prédiabète, diabète de type 1 ou de type 2, résistance à l'insuline, acromégalie, diabète avec concentration élevée d'IGF-1, obésité, hyperglycémie, intolérance au glucose, hyperinsulinémie, dyslipidémie, dysadipokinémie, inflammation subclinique, inflammation systémique, inflammation systémique de bas grade, lipidose hépatique, athérosclérose, inflammation du pancréas, neuropathie et/ou syndrome X (syndrome métabolique) et/ou perte de la fonction des cellules bêta pancréatiques et/ou dans laquelle la rémission du trouble métabolique, de préférence la rémission diabétique, est obtenue et/ou maintenue ;
(iv) un trouble métabolique d'un animal canin, dans laquelle de préférence le trouble métabolique est l'un ou plusieurs choisis dans le groupe constitué par : acidocétose, prédiabète, diabète insulinodépendant, diabète insulinorésistant, insulinorésistance, obésité, hyperglycémie, cataracte induite par l'hyperglycémie, intolérance au glucose, hyperinsulinémie, dyslipidémie, dysadipokinémie, inflammation subclinique, inflammation systémique, inflammation systémique de bas grade, lipidose hépatique, pancréatite, conséquences de troubles métaboliques, telles que l'hypertension, l'insuffisance rénale et/ou les troubles musculo-squelettiques, et/ou syndrome X (syndrome métabolique), de préférence prédiabète, diabète insulinodépendant, diabète insulinorésistant, insulinorésistance, dans lequel de préférence le développement de la cataracte induite par l'hyperglycémie est prévenu ou une rémission est obtenue et/ou dans lequel le développement des conséquences de troubles métaboliques, telles que l'hypertension, l'insuffisance rénale et/ou les troubles musculo-squelettiques, est prévenu ou la progression est ralentie ou une rémission est obtenue ;
(v) une maladie cardiaque d'un félin, de préférence dans laquelle la maladie cardiaque est l'une ou plusieurs choisies dans le groupe constitué par : insuffisance cardiaque, insuffisance cardiaque due à une ou plusieurs cardiomyopathies, insuffisance cardiaque due à une cardiomyopathie hypertrophique (CMH), insuffisance cardiaque due à une cardiomyopathie restrictive (CMR), insuffisance cardiaque due à une cardiomyopathie dilatée (CMD), insuffisance cardiaque due à une cardiomyopathie non classée (CNC), insuffisance cardiaque due à une cardiomyopathie arythmogène du ventricule droit (CAVD), cardiomyopathie hypertrophique (CMH), cardiomyopathie restrictive (CMR), cardiomyopathie dilatée (CMD), cardiomyopathie non classée (CNC) et/ou cardiomyopathie arythmogène du ventricule droit (CAVD) ; de préférence choisie dans le groupe constitué par : insuffisance cardiaque due à une ou plusieurs cardiomyopathies, insuffisance cardiaque due à une cardiomyopathie hypertrophique (CMH), cardiomyopathie hypertrophique (CMH) ;
(vi) le dessèchement d'un mammifère non humain, de préférence un ruminant, l'amélioration et/ou la facilitation du dessèchement d'un mammifère non humain, de préférence un ruminant, la réduction la production de lait, de préférence la production et/ou la sécrétion de lait, chez un mammifère non humain gravide et/ou allaitant, de préférence un ruminant, la diminution de l'accumulation de lait et/ou l'engorgement dans la mamelle, de préférence la mamelle et/ou la glande mammaire, d'un mammifère non humain, de préférence un ruminant, la diminution de l'inconfort associé à l'engorgement de la mamelle, tel que l'augmentation du temps de couchage quotidien et/ou la réduction du stress, d'un mammifère non humain, de préférence un ruminant, la diminution des fuites de lait après le dessèchement d'un mammifère non humain, de préférence un ruminant, la diminution de l'incidence des infections intramammaires (IMI), de préférence la mammite et/ou la métrite, chez un mammifère non humain, de préférence un ruminant ;
(vii) une maladie cardiaque d'un mammifère non humain, à l'exclusion d'un félin, en particulier d'un canidé, dans laquelle de préférence la maladie cardiaque est l'une ou plusieurs choisies dans le groupe constitué par : insuffisance cardiaque ; insuffisance cardiaque congestive ; insuffisance cardiaque asymptomatique/préclinique/occulte ; insuffisance cardiaque due à une maladie de la valve mitrale (myxomateuse) [(M)MVD] ; insuffisance cardiaque congestive due à une maladie de la valve mitrale (myxomateuse) [(M)MVD] ; insuffisance cardiaque asymptomatique/préclinique/occulte due à une maladie de la valve mitrale (myxomateuse) [(M)MVD] ; maladie de la valve mitrale (myxomateuse) [(M)MVD] ; maladie de la valve mitrale (myxomateuse) [(M)MVD] cliniquement manifeste ; maladie de la valve mitrale (myxomateuse) [(M)MVD] asymptomatique/préclinique/occulte ; insuffisance cardiaque due à une cardiomyopathie dilatée (CMD) ; insuffisance cardiaque congestive due à une cardiomyopathie dilatée (CMD) ; insuffisance cardiaque asymptomatique/préclinique/occulte due à une cardiomyopathie dilatée (CMD) ; cardiomyopathie dilatée (CMD) ; cardiomyopathie dilatée (CMD) cliniquement manifeste ; cardiomyopathie dilatée (CMD) asymptomatique/préclinique/occulte ; sténose aortique (valvulaire, supravalvulaire et/ou sous-valvulaire) ;
(viii) hypertension chez un mammifère non humain, de préférence un carnivore, plus préférablement un chat ou un chien, dans laquelle l'hypertension est de préférence l'une ou plusieurs choisies dans le groupe constitué par : hypertension situationnelle, hypertension secondaire et hypertension idiopathique, dans laquelle l'hypertension secondaire est de préférence choisie parmi les hypertensions associées à une maladie rénale chronique (MRC), au diabète, à l'obésité, aux maladies cardiaques, aux maladies endocriniennes, telles que la maladie de Cushing, l'hyperthyroïdie, l'acromégalie et l'élévation de la pression artérielle (PA) induite par des médicaments, de préférence par des glucocorticoïdes, des minéralocorticoïdes, des agents stimulant l'érythropoïèse, l'éphédrine et/ou du chlorure de sodium à forte dose ;
(ix) une maladie rénale d'un mammifère non humain, de préférence un carnivore, plus préférablement un chat ou un chien, dans laquelle de préférence la maladie rénale est l'une ou plusieurs choisies dans le groupe constitué par : dysplasie rénale, glomérulopathie, polykystose rénale, amylose, tubulo-néphrite/néphrite tubulo-interstitielle (NTI), maladie rénale aiguë, maladie rénale chronique.

20. Procédé de production de la composition pharmaceutique aqueuse selon l'une quelconque des revendications 1 à 18, comprenant les étapes (dans n'importe quel ordre significatif) :
(i) de l'eau est utilisée comme matière première ;
(ii) ajout d'un ou plusieurs agents solubilisants à l'eau issue de l'étape (i) ;
(iii) dissolution de vélagliflozine dans le mélange issu de l'étape (ii) ;
(iv) éventuellement, dissolution du ou des conservateurs dans le mélange issu de l'étape (iii) ;
(v) éventuellement, en outre dissolution d'autres excipients, tels qu'un ou plusieurs agent(s) modificateur(s) de pH, un ou plusieurs arôme(s), des édulcorants, des agents anti-oxydation, des agents d'amélioration de la viscosité et analogues, dans le mélange issu de l'étape (iii) ou éventuellement (iv) ;
(vi) éventuellement, filtration du mélange issu de l'étape (iii), éventuellement de l'étape (iv) ou éventuellement de l'étape (v) ;
ce faisant, éventuellement, indépendamment les unes des autres après l'une quelconque des étapes individuelles du procédé - qu'elles soient obligatoires ou facultatives - une étape de mélange supplémentaire est réalisée.

21. Kit de pièces comprenant :
(a) une composition pharmaceutique aqueuse selon l'une quelconque des revendications 1 à 18 ; et
(b) une notice d'emballage comprenant les informations indiquant que la composition pharmaceutique aqueuse doit être utilisée pour la prévention et/ou le traitement d'une ou plusieurs indications médicales chez un sujet nécessitant une telle prévention et/ou un tel traitement, qui sont choisies parmi les indications médicales :
(i) un trouble métabolique d'un équidé, de préférence dans laquelle le trouble métabolique est un ou plusieurs troubles choisis parmi la résistance à l'insuline, l'hyperinsulinémie, l'intolérance au glucose, la dyslipidémie, la dysadipokinémie, l'inflammation subclinique, l'inflammation systémique, l'inflammation systémique de bas grade, l'obésité et/ou l'adiposité régionale, de préférence une résistance à l'insuline, une hyperinsulinémie et/ou une affection clinique associée à la résistance à l'insuline et/ou à l'hyperinsulinémie ; de préférence ladite affection clinique étant une ou plusieurs affections choisies parmi l'intolérance au glucose, la dyslipidémie, la dysadipokinémie, l'inflammation subclinique, l'inflammation systémique, l'inflammation systémique de bas grade, l'obésité et/ou l'adiposité régionale ;
(ii) un trouble métabolique d'un équidé, dans laquelle le trouble métabolique est un ou plusieurs troubles choisis parmi la fourbure, le dysfonctionnement vasculaire, l'hypertension, la lipidose hépatique, l'athérosclérose, l'hyperadrénocorticisme, le dysfonctionnement de la pars intermedia de l'hypophyse et/ou le syndrome métabolique équin, de préférence le trouble métabolique étant un état/signe clinique associé à une résistance à l'insuline et/ou à une hyperinsulinémie, ledit état/signe clinique étant de préférence un ou plusieurs états choisis parmi la fourbure, le dysfonctionnement vasculaire, l'hypertension, la lipidose hépatique, l'athérosclérose, l'hyperadrénocorticisme, le dysfonctionnement de la pars intermedia de l'hypophyse et/ou le syndrome métabolique équin ;
(iii) un trouble métabolique d'un animal félin, dans laquelle de préférence le trouble métabolique est l'un ou plusieurs choisis dans le groupe constitué par : acidocétose, prédiabète, diabète de type 1 ou de type 2, résistance à l'insuline, acromégalie, diabète avec concentration élevée d'IGF-1, obésité, hyperglycémie, intolérance au glucose, hyperinsulinémie, dyslipidémie, dysadipokinémie, inflammation subclinique, inflammation systémique, inflammation systémique de bas grade, lipidose hépatique, athérosclérose, inflammation du pancréas, neuropathie et/ou syndrome X (syndrome métabolique) et/ou perte de la fonction des cellules bêta pancréatiques et/ou dans laquelle la rémission du trouble métabolique, de préférence la rémission diabétique, est obtenue et/ou maintenue ;
(iv) un trouble métabolique d'un animal canin, dans laquelle de préférence le trouble métabolique est l'un ou plusieurs choisis dans le groupe constitué par : acidocétose, prédiabète, diabète insulinodépendant, diabète insulinorésistant, insulinorésistance, obésité, hyperglycémie, cataracte induite par l'hyperglycémie, intolérance au glucose, hyperinsulinémie, dyslipidémie, dysadipokinémie, inflammation subclinique, inflammation systémique, inflammation systémique de bas grade, lipidose hépatique, pancréatite, conséquences de troubles métaboliques, telles que l'hypertension, l'insuffisance rénale et/ou les troubles musculo-squelettiques, et/ou syndrome X (syndrome métabolique), de préférence prédiabète, diabète insulinodépendant, diabète insulinorésistant, insulinorésistance, dans lequel de préférence le développement de la cataracte induite par l'hyperglycémie est prévenu ou une rémission est obtenue et/ou dans lequel le développement des conséquences de troubles métaboliques, telles que l'hypertension, l'insuffisance rénale et/ou les troubles musculo-squelettiques, est prévenu ou la progression est ralentie ou une rémission est obtenue ;
(v) une maladie cardiaque d'un félin, de préférence dans laquelle la maladie cardiaque est l'une ou plusieurs choisies dans le groupe constitué par : insuffisance cardiaque, insuffisance cardiaque due à une ou plusieurs cardiomyopathies, insuffisance cardiaque due à une cardiomyopathie hypertrophique (CMH), insuffisance cardiaque due à une cardiomyopathie restrictive (CMR), insuffisance cardiaque due à une cardiomyopathie dilatée (CMD), insuffisance cardiaque due à une cardiomyopathie non classée (CNC), insuffisance cardiaque due à une cardiomyopathie arythmogène du ventricule droit (CAVD), cardiomyopathie hypertrophique (CMH), cardiomyopathie restrictive (CMR), cardiomyopathie dilatée (CMD), cardiomyopathie non classée (CNC) et/ou cardiomyopathie arythmogène du ventricule droit (CAVD) ; de préférence choisie dans le groupe constitué par : insuffisance cardiaque due à une ou plusieurs cardiomyopathies, insuffisance cardiaque due à une cardiomyopathie hypertrophique (CMH), cardiomyopathie hypertrophique (CMH) ;
(vi) le dessèchement d'un mammifère non humain, de préférence un ruminant, l'amélioration et/ou la facilitation du dessèchement d'un mammifère non humain, de préférence un ruminant, la réduction la production de lait, de préférence la production et/ou la sécrétion de lait, chez un mammifère non humain gravide et/ou allaitant, de préférence un ruminant, la diminution de l'accumulation de lait et/ou l'engorgement dans la mamelle, de préférence la mamelle et/ou la glande mammaire, d'un mammifère non humain, de préférence un ruminant, la diminution de l'inconfort associé à l'engorgement de la mamelle, tel que l'augmentation du temps de couchage quotidien et/ou la réduction du stress, d'un mammifère non humain, de préférence un ruminant, la diminution des fuites de lait après le dessèchement d'un mammifère non humain, de préférence un ruminant, la diminution de l'incidence des infections intramammaires (IMI), de préférence la mammite et/ou la métrite, chez un mammifère non humain, de préférence un ruminant ;
(vii) une maladie cardiaque d'un mammifère non humain, à l'exclusion d'un félin, en particulier d'un canidé, dans laquelle de préférence la maladie cardiaque est l'une ou plusieurs choisies dans le groupe constitué par : insuffisance cardiaque ; insuffisance cardiaque congestive ; insuffisance cardiaque asymptomatique/préclinique/occulte ; insuffisance cardiaque due à une maladie de la valve mitrale (myxomateuse) [(M)MVD] ; insuffisance cardiaque congestive due à une maladie de la valve mitrale (myxomateuse) [(M)MVD] ; insuffisance cardiaque asymptomatique/préclinique/occulte due à une maladie de la valve mitrale (myxomateuse) [(M)MVD] ; maladie de la valve mitrale (myxomateuse) [(M)MVD] ; maladie de la valve mitrale (myxomateuse) [(M)MVD] cliniquement manifeste ; maladie de la valve mitrale (myxomateuse) [(M)MVD] asymptomatique/préclinique/occulte ; insuffisance cardiaque due à une cardiomyopathie dilatée (CMD) ; insuffisance cardiaque congestive due à une cardiomyopathie dilatée (CMD) ; insuffisance cardiaque asymptomatique/préclinique/occulte due à une cardiomyopathie dilatée (CMD) ; cardiomyopathie dilatée (CMD) ; cardiomyopathie dilatée (CMD) cliniquement manifeste ; cardiomyopathie dilatée (CMD) asymptomatique/préclinique/occulte ; sténose aortique (valvulaire, supravalvulaire et/ou sous-valvulaire) ;
(viii) hypertension chez un mammifère non humain, de préférence un carnivore, plus préférablement un chat ou un chien, dans laquelle l'hypertension est de préférence l'une ou plusieurs choisies dans le groupe constitué par : hypertension situationnelle, hypertension secondaire et hypertension idiopathique, dans laquelle l'hypertension secondaire est de préférence choisie parmi les hypertensions associées à une maladie rénale chronique (MRC), au diabète, à l'obésité, aux maladies cardiaques, aux maladies endocriniennes, telles que la maladie de Cushing, l'hyperthyroïdie, l'acromégalie et l'élévation de la pression artérielle (PA) induite par des médicaments, de préférence par des glucocorticoïdes, des minéralocorticoïdes, des agents stimulant l'érythropoïèse, l'éphédrine et/ou du chlorure de sodium à forte dose ;
(ix) une maladie rénale d'un mammifère non humain, de préférence un carnivore, plus préférablement un chat ou un chien, dans laquelle de préférence la maladie rénale est l'une ou plusieurs choisies dans le groupe constitué par : dysplasie rénale, glomérulopathie, polykystose rénale, amylose, tubulo-néphrite/néphrite tubulo-interstitielle (NTI), maladie rénale aiguë, maladie rénale chronique.
